(19) Europäisches Patentamt
European Patent Office
Office européen des brevets

(11) **EP 3 027 252 B1**

(12) **EUROPÄISCHE PATENTSCHRIFT**

(45) Veröffentlichungstag und Bekanntmachung des Hinweises auf die Patenterteilung:
**25.04.2018 Patentblatt 2018/17**

(21) Anmeldenummer: **14739354.0**

(22) Anmeldetag: **07.07.2014**

(51) Int Cl.:
*A61M 16/00* (2006.01)   *A61B 5/08* (2006.01)

(86) Internationale Anmeldenummer:
**PCT/EP2014/001863**

(87) Internationale Veröffentlichungsnummer:
**WO 2015/007373 (22.01.2015 Gazette 2015/03)**

(54) **MEDIZINISCHE MESSVORRICHTUNG, BEATMUNGSVORRICHTUNG UND VERFAHREN ZUM BETRIEB EINER MEDIZINISCHEN MESSVORRICHTUNG**

MEDICAL MEASURING DEVICE, VENTILATION DEVICE AND METHOD FOR OPERATING A MEDICAL MEASURING DEVICE

DISPOSITIF DE MESURE MÉDICAL, APPAREIL RESPIRATOIRE ET PROCÉDÉ POUR FAIRE FONCTIONNER UN DISPOSITIF DE MESURE MÉDICAL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **18.07.2013 DE 102013011983**

(43) Veröffentlichungstag der Anmeldung:
**08.06.2016 Patentblatt 2016/23**

(73) Patentinhaber: **Drägerwerk AG & Co. KGaA 23558 Lübeck (DE)**

(72) Erfinder:
• HEESCH, Ralf
  23568 Lübeck (DE)
• BRANDT, Andreas
  23619 Hamberge (DE)

(56) Entgegenhaltungen:
WO-A1-2013/068918   WO-A2-2007/023492
WO-A2-2010/058308

**Beschreibung**

Die vorliegende Erfindung betrifft eine medizintechnische Messvorrichtung, eine Beatmungsvorrichtung mit einer medizintechnischen Messvorrichtung, ein Verfahren zum Betrieb einer medizintechnischen Messvorrichtung, sowie ein Verfahren zum Betrieb einer Beatmungsvorrichtung mit einer medizintechnischen Messvorrichtung.

Medizintechnische Messvorrichtungen sind bekannt zur messtechnischen Überprüfung von Medizingeräten, insbesondere zur Überprüfung eines zuverlässigen und sicheren Betriebes der Medizingeräte im Einsatz an einem Patienten.

Unter einer Beatmungsvorrichtung kann in diesem Zusammenhang beispielsweise ein Beatmungsgerät oder ein Anästhesiegerät verstanden werden.

Im klinischen Umfeld kann eine Beatmung eines Menschen oder eines Lebewesens durch eine Beatmungsvorrichtung vorgenommen werden.

Zu einem Einsatz auf einer Intensivstation kann eine solche Beatmungsvorrichtung als ein Beatmungsgerät ausgeführt sein. Zu einer Verwendung im Umfeld einer Operation kann eine solche Beatmungsvorrichtung auch ein Anästhesiegerät ein. Denkbar ist auch, dass eine solche Beatmungsvorrichtung für eine Anwendung bei Notfalleinsätzen als ein Notfallbeatmungsgerät ausgeführt ist. Bei der Beatmung wird die in der Lunge vorhandene Atemluft zyklisch im Atemtakt einer Beatmungsfrequenz ausgetauscht. Neben den Atemgasen ist in der Lunge und im Bronchialtrakt eines Patienten zusätzlich Sekret vorhanden. Bei einer spontanen Atemtätigkeit wird dieses in der Lunge vorhandene Sekret, der sogenannte Mucus, im Regelfall durch die normale natürliche Ausatmung aus der Lunge und dem Bronchialtrakt herausgefördert.

Diese Förderung hinaus aus der Lunge und dem Bronchialtrakt wird üblicherweise durch weitere, durch Reize hervorgerufene Reaktionen eines Patienten, wie Husten oder Niesen unterstützt.

Bei einer maschinellen Beatmung durch eine Beatmungsvorrichtung wird der Patient im Regelfall mittels eines Endotrachialtubus oder einer Nasalmaske beatmet. An den Endotrachialtubus oder die Nasalmaske schließt sich ein Beatmungsschlauch oder ein Beatmungsschlauchsystem zu einer luft- und gasführenden Verbindung an die Beatmungsvorrichtung an. Bei maschineller Beatmung wird die Atmung eines Patienten d. h. der Wechsel zwischen Ein- und Ausatmung durch Einstellungen an der Beatmungsvorrichtung im Wesentlichen mandatorisch, d.h. zwangsgeführt und automatisiert, vorgegeben.

Die Einstellungen von Beatmungsparametern an der Beatmungsvorrichtung wie Beatmungsfrequenz (RR- Rate), Inspiration- zu Exspirations- Zeitverhältnis (I:E-Ratio), inspiratorischer Druck und Druckverlauf, end-exspiratorischer Druck (PEEP), exspiratorischer Druck und Druckverlauf, Durchfluss-Obergrenzen, Druck-Obergrenzen, Atemminutenvolumen (AMV) sind üblicherweise an Beatmungsformen oder Beatmungsmodi gekoppelt. Beatmungsvorrichtungen nach dem Stand der Technik ermöglichen üblicherweise verschiedene Formen von "Druck- Kontrollierten" und "Volumen- Kontrollierten" Beatmungsformen. Beatmungsgeräte zur Durchführung einer maschinellen Beatmung nach dem Stand der Technik sind in US 2,904,035, US 5,400,777, US 5,937,853,

WO 2007/085110 A1 beschrieben. Anästhesiegeräte zur Durchführung einer Narkose an menschlichen oder tierischen Lebewesen nach dem Stand der Technik sind in der WO 2008/098382 A1, US 6,571,792, US 6,553,990 beschrieben. Ein Verfahren zur Bestimmung eines Qualitätsmaßes einer Beatmung anhand einer Auswertung der durch einen Patienten verrichteten Atemarbeit ist aus der WO 2010/022513A1 bekannt.

Die Einstellungen des Beatmungsgerätes haben in diesem Zusammenhang typischerweise nicht nur Auswirkungen auf den Gasaustausch selbst, sondern können sich auch auf den Transport des Sekrets auswirken. Dabei kann es auch zu einer Ansammlung von Sekret in der Lunge eines Patienten kommen. In der klinisch-medizinischen Literatur, wie in "Respiratory Care, October 2008, Vol. 53 No.10", ist der prinzipielle Einfluss von Beatmungseinstellungen auf den Transport des Sekrets beschrieben. Solche Sekret- Ansammlung in der Lunge können den Gasaustausch in der Lunge und damit verbunden auch den Austausch von $CO_2$ und $O_2$ im Blut nachteilig beeinflussen. Dieses Problem wird typischerweise im klinischen Alltag dadurch gelöst, dass das klinische Personal im Rahmen der klinischen Routine in regelmäßigen Abständen eine sogenannte SekretAbsaugung durchgeführt. Dazu wird der Patient kurzzeitig vom Beatmungsgerät diskonnektiert, d.h. die luft- oder gasführende Verbindung zwischen der Beatmungsvorrichtung und dem Patienten unterbrochen und über den Endotrachialtubus wird mittels einer Sekret- Absaugeeinrichtung das Sekret aus der Lunge abgesaugt.

Eine solche Sekret- Absaugeeinrichtung ist in der US 5,606,968 gezeigt.

Die übliche klinische Routine orientiert sich dabei im Wesentlichen an Erfahrungswerten hinsichtlich der Häufigkeit der Anwendung der Sekretabsaugung und hinsichtlich der Zeitabstände zwischen den Sekretabsaugungen im Tagesverlauf. Zusätzlich können die individuellen Krankheitsbilder der Patienten, sowie deren Konstitution und Alter in die Routine mit einbezogen werden.

Die gewählten Beatmungsformen und/oder gewählten Beatmungsparameter am Beatmungsgerät können einen Einfluss auf die Verteilung von Sekret in Bronchialtakt oder Lunge eines Patienten haben. Weiterhin können die gewählten Beatmungsformen und/oder Beatmungsparameter die Mengen- Verteilung des Sekrets innerhalb des Bronchialbereiches (oberer Bronchialtrakt, unterer Bronchialbereich, Lunge) beeinflussen. Die Vielfalt der Wechselwirkungen zwischen den Einstellungen am Beatmungsgerät und der persönlichen Konstitution und den Krankheitsbildern der verschiedenen Patienten kann bedingen, dass in der klinischen Routine die Durchführungen von Sekretabsaugungen mit einem zeitlichen Abstand erfolgen, welche im klinischen Alltag nicht immer an die individuellen Erfordernisse der Patienten angepasst sein müssen. Aus der (WO2010/058308 A2) ist eine Vorrichtung zur Entfernung von Sekret im Bronchialtrakt bekannt, mittels einer Erhöhung und Absenkung eines Beatmungsdrucks Sekret im Bronchialtrakt aufwärts zu fördern.

Die Situation der Sekretabsaugung stellt in den meisten Fällen für den Patienten sowohl einen unbequemen, teilweise schmerzhaften, in jedem Fall aber die Konstitution und das allgemeine Wohlbefinden belastenden Vorgang dar. Hinzu kommt, dass der diskonnektierte Patient für die Dauer der Absaugung kurzzeitig nicht beatmet werden kann.

Es ist die Aufgabe der vorliegenden Erfindung, diesen und anderen Nachteilen des Standes der Technik zu begegnen. Insbesondere soll eine Vorrichtung bereitgestellt werden, mit der bei einer künstlichen Beatmung eines Patienten die Verlagerung von Sekret in die Lunge, insbesondere in untere und tiefere Bereiche der Lunge, möglichst gering gehalten werden kann.

Weiterhin ist es eine Aufgabe der vorliegenden Erfindung, ein Verfahren zum Betrieb einer Vorrichtung mit einer verringerten Verlagerung von Sekret in die Lunge, insbesondere in untere und tiefere Bereiche der Lunge, anzugeben.

Als Lösung dieser und weiterer Aufgaben sieht die Erfindung eine Vorrichtung mit den Merkmalen des Patentanspruches 1, eine Vorrichtung mit den Merkmalen des Patentanspruches 7 und ein Verfahren mit den Merkmalen des Patentanspruches 13, vor.

Eine weitere erfindungsgemäße Lösung sieht eine gemeinsame Verwendung der Vorrichtungen gemäß Anspruch 1 und Anspruch 7 mit den Merkmalen von Anspruch 16 vor. Vorteilhafte Ausgestaltungen ergeben sich aus den Unteransprüchen.

Eine erfindungsgemäße Vorrichtung weist eine Sensorik und Mittel zur Messwerterfassung, Signalverarbeitung und Berechnung auf, wobei die Sensorik ausgebildet ist,

- eine inspiratorische Messgröße zu erfassen,
  welche ein Maß für einen Transport von Atemgasen
  in eine Lunge eines Patienten hinein darstellt
  und wobei die Sensorik ausgebildet ist,
- eine exspiratorische Messgröße zu erfassen,
  welche ein Maß für einen Transport von Atemgasen
  aus der Lunge eines Patienten hinaus darstellt
  und wobei die Mittel zur Messwerterfassung, Signalverarbeitung und Berechnung dazu ausgebildet sind,
- aus der exspiratorischen Messgröße und der
  inspiratorischen Messgröße ein Maß aus einem Quotienten aus der exspiratorischen Messgröße und der inspiratorischen Messgröße für eine beatmungsbedingte Verlagerung von Sekret zu bestimmen.

Die Sensorik ist ausgebildet, eine inspiratorische Messgröße zu erfassen, welche ein Maß für einen Transport von Atemgasen in die Lunge eines Patienten hinein darstellt. Die Sensorik ist weiterhin ausgebildet, eine exspiratorische Messgröße zu erfassen, welche ein Maß für einen Transport von Atemgasen aus der Lunge eines Patienten hinaus darstellt. Die Sensorik kann im Sinne der vorliegenden Erfindung aus einem Sensor oder aus mehreren Sensoren bestehen, die so in oder an der Vorrichtung angeordnet oder mit dieser verbunden sind, Luft- oder Gasbewegungen in die Lunge hinein oder aus der Lunge hinaus zu erfassen und den Mitteln zur Messwerterfassung, Signalverarbeitung und Berechnung als Messdaten zur Verfügung zu stellen. Die Sensorik kann vorzugsweise aus einem oder mehreren Durchflusssensoren bestehen.

Unter einer Erfassung kann typischerweise im Sinne der vorliegenden Erfindung jegliche Art einer Erfassung einer Zustandsgröße, einer physikalischen, elektrischen oder chemischen Messgröße oder eines die Messgröße repräsentierenden elektrischen Signals in Form einer elektrischen Spannung, elektrischen Stromes oder elektrischen Widerstandes verstanden werden.

Inspiratorische Messgrößen können im Sinne der vorliegenden Erfindung von der Sensorik erfasste Messgrößen sein, die für Luft- oder Gasbewegungen, oder Luft-oder Gasmengen in die Lunge hinein qualitativ und/oder quantitativ kennzeichnend sind. Insbesondere eine inspiratorische Durchflussmenge stellt im Sinne der vorliegenden Erfindung eine bevorzugte Ausbildung einer inspiratorischen Messgröße dar.

Exspiratorische Messgrößen können im Sinne der vorliegenden Erfindung von der Sensorik erfasste Messgrößen sein, die für Luft- oder Gasbewegungen, oder Luft-oder Gasmengen aus der Lunge hinaus qualitativ und/oder quantitativ kennzeichnend sind. Insbesondere eine exspiratorische Durchflussmenge stellt im Sinne der vorliegenden Erfindung eine bevorzugte Ausbildung einer exspiratorischen Messgröße dar.

Die Mittel zur Messwerterfassung, Signalverarbeitung und Berechnung sind vorteilhaft dazu ausgebildet, Messwerte, Signale oder Daten der Sensorik oder anderer Datenquellen zu erfassen oder aufzunehmen, umzuwandeln, weiterzuverarbeiten und/oder Berechnungen und/oder Umrechnungen mit diesen Messwerten, Signalen oder Daten durchzuführen.

Die Mittel zur Messwerterfassung, Signalverarbeitung und Berechnung sind vorzugsweise als Kombination oder Kombinationen von Mitteln zur Messwerterfassung, Mitteln zur Signalverarbeitung und Mitteln zur Berechnung gemeinsam in einer Einheit zur Messwerterfassung, Signalverarbeitung und Berechnung ausgeführt. In einer alternativen Ausgestaltung können die Mittel zur Messwerterfassung, Mittel zur Signalverarbeitung und Mittel zur Berechnung auch in einzelnen Baugruppen, Kombinationen einzelner Baugruppen oder als Bestandteile anderer und/oder weiterer Baugruppen ausgestaltet sein.

Unter einer Verarbeitung, Umwandlung, Berechnung oder Umrechnung von Messwerten, Signalen, Daten ist im Sinne der vorliegenden Erfindung jegliche Art von Signalanpassung, Signalvorverstärkung, Signalverstärkung, analoger oder digitaler Filterung, Analog- zu- Digitalwandlung, Digital- zu- Analog- Wandlung, mathematischen Berechnungen oder Umrechnungen zu verstehen. Unter dem Begriff einer Sensorik ist im Sinne der vorliegenden Erfindung eine Umwandlung von physikalischen oder chemischen Messgrößen, wie Druck, Druckdifferenz, Partialdruck, Temperatur, Feuchtigkeit, Stoff-Konzentration, Geschwindigkeit, Strömungsgeschwindigkeit, Durchflussgeschwindigkeit, Durchflussmenge, Volumen, Zeit in ein elektrisches Spannungs-, Strom oder Widerstandsäquivalent zu verstehen.

Weiterhin ist im Sinne der vorliegenden Erfindung auch vorstellbar, dass in der Sensorik neben einem sensorischen Element zur Messung einer physikalischen oder chemischen Messgröße Elemente, Teile der Mittel oder Einheit zur Messwerterfassung, Signalverarbeitung und Berechnung, wie Erfassung, Verarbeitung, Umwandlung, wie beispielsweise Spannungsmessung, Strommessung, Widerstandsmessung, Signalanpassung, Signalvorverstärkung, Signalverstärkung, analoger oder digitaler Filterung, Analog- zu- Digitalwandlung, Digital- zu- Analog- Wandlung integriert sein können.

Die Mittel zur Messwerterfassung, Signalverarbeitung und Berechnung sind weiterhin ausgebildet, ein Maß für eine beatmungsbedingte Verlagerung von Sekret aus der inspiratorischen Messgröße, welche ein Maß für den Transport von Atemgasen in die Lunge eines Patienten hinein darstellt und aus der exspiratorischen Messgröße, welche ein Maß für den Transport von Atemgasen aus der Lunge eines Patienten hinaus darstellt, zu bestimmen.

Das Maß für ein beatmungsbedingte Verlagerung von Sekret kann im Sinne der vorliegenden Erfindung ein aus der exspiratorischen Messgröße und der inspiratorischen Messgröße bestimmter, abgeleiteter, prognostizierter, abgeschätzter oder berechneter Wert sein, der eine Kennzahl, einen Index ein abgeleitetes oder berechnetes Verhältnis, einen mathematischen Zusammenhang oder eine Relation dahingehend darstellt, in welcher Weise eine beatmungsbedingte Verlagerung von Sekret vorliegt.

Erfindungsgemäß wird das Maß für eine beatmungsbedingte Verlagerung von Sekret als ein Quotient der inspiratorischen Messgröße zur exspiratorischen Messgröße gebildet oder von diesem Quotienten abgeleitet. Das Verhältnis der inspiratorischen Messgröße zur exspiratorischen Messgröße kann dabei in mathematischer Form aus einem Quotienten eines Messwertes oder einer Folge von Messwerten der inspiratorischen Messgröße zu einem Messwert oder einer Folge von Messwerten der exspiratorischen Messgröße gebildet werden.

Aus der vorliegenden Erfindung ergeben sich unter anderem folgende Vorteile für den Betrieb von Medizingeräten, insbesondere von Beatmungsgeräten oder Anästhesiegeräten. Das Maß für eine beatmungsbedingte Verlagerung von Sekret ermöglicht Anpassungen von Einstellungen am Beatmungsgerät oder Anästhesiegeräten zur Beatmung eines Patienten. Diese Anpassung der Beatmungseinstellungen kann eine für den Patienten hinsichtlich Häufigkeit und Aufwand der Sekretabsaugungen komfortablere Situation ermöglichen. Die Bereitstellung des Maßes für eine beatmungsbedingte Verlagerung von Sekret durch die vorliegende Erfindung ermöglicht eine geeignete Anpassung der Beatmungseinstellungen in Bezug auf die Verteilung von Sekret im Bronchialtrakt und auf die Ansammlung von Sekret in der Lunge. Daraus können sich in der Folge für einen Patienten in vorteilhafter Weise eine Verringerung der Häufigkeit von Sekretabsaugungen, eine Minimierung des Aufwandes bei den Sekretabsaugungen, eine Verringerung der für die jeweiligen Sekretabsaugungen erforderlichen Zeitdauern, sowie eine Verringerung der Anzahl und/oder Zeitdauern von Diskonnektierungen ergeben. Insgesamt ist es so möglich, dass sich mit Hilfe der vorliegenden Erfindung weniger Belastungen auf die Konstitution und das allgemeine Wohlbefinden eines Patienten ergeben können. Ohne eine Unterstützung durch das Maß für eine beatmungsbedingte Verlagerung von Sekret ist es für den Anwender, insbesondere wegen der Vielfalt der möglichen Wechselwirkungen zwischen Beatmungsmodi, Beatmungsparametern und der individuellen Gegebenheit eines Patienten oftmals schwierig, lediglich auf Basis von angezeigten Messwerten oder Diagrammen am Beatmungsgerät, wie beispielsweise aus einem Verlauf des Beatmungsdrucks oder dem Verlauf der Durchflussmenge einschätzen zu können, in welcher Weise die gerade aktuell gewählte Beatmung eine Ansammlung von Sekret in der Lunge, insbesondere in tieferen Lungenbereichen, begünstigt oder weniger begünstigt.

In einer bevorzugten Ausführungsform der Medizintechnischen Messvorrichtung sind die Mittel zur Messwerterfassung, Signalverarbeitung und Berechnung ausgebildet, einen Vergleich des Maßes für die beatmungsbedingte Verlagerung von Sekret mit einem vorbestimmten Wert durchzuführen.

Der vorbestimmte Wert kann bevorzugt als ein Schwellwert ausgebildet sein. Durch einen Vergleich des zuvor bestimmten Maßes für eine beatmungsbedingte Verlagerung von Sekret mit dem vorbestimmten Wert oder mit dem Schwellwert kann ermittelt werden, ob bei der Durchführung der Beatmung ein inspiratorischer Impuls, d.h. ein Transport von Sekret in die Lunge hinein, oder ein exspiratorischer Impuls, d.h. ein Transport von Sekret aus der Lunge hinaus, überwiegt.

Stehen der inspiratorische Impuls und der exspiratorische Impuls in einem Gleichgewicht zueinander, so ergibt sich aus dem Quotienten des inspiratorischen Impulses und des exspiratorischen Impulses mathematisch ein Sekret-Index-Wert ($S_i$) von 1.0, bei dem im Regelfall keine Verlagerung von Sekret stattfindet. Der Sekret-Index-Wert ($S_i$) von 1.0 kann damit vorzugsweise als eine mittlere Bezugs-und Vergleichsgröße für die beatmungsbedingte Verlagerung von Sekret und/ oder als ein möglicher vorbestimmter Wert oder möglicher Schwellwert dienen. In bevorzugter Weise kann der vorbestimmte Wert auch als ein Schwellwertbereich mit einem oberen und einem unterem Schwellwert ausgeführt sein, so dass sich zwischen dem unteren und dem oberen Schwellwert eine Hysterese ergibt. Weiter bevorzugt ist der Bereich der Hysterese ein Sollbereich des Maßes für eine beatmungsbedingte Verlagerung von Sekret. Im Sinne der vorliegenden Erfindung sind dabei unter den Begriffen des vorbestimmten Wertes oder des Schwellwertes auch Schwellwertbereiche mit zu verstehen, so dass Vergleiche des Maßes für eine beatmungsbedingte Verlagerung von Sekret mit einem vorbestimmten Wert beispielsweise eine Überschreitung des oberen Schwellwertes, sowie eine Unterschreitung des unteren Schwellwertes eine Abweichung vom Sollbereich des Maßes für eine beatmungsbedingte Verlagerung von Sekret darstellen. Eine Unterschreitung des oberen Schwellwertes und gleichzeitige Überschreitung des unteren Schwellwertes stellen hingegen eine Situation dar, in welcher sich das Maß für eine beatmungsbedingte Verlagerung von Sekret im Sollbereich befindet. Der vorbestimmte Wert oder Schwellwert ($S_{Basis}$) wird vorzugsweise im Bereich von 0,8 bis 1,2 angesetzt.

Falls der inspiratorische Impuls ($S_i$ >1.0) überwiegt, so wird bedingt durch die Art und Weise der Beatmung und die dafür ursächlichen Beatmungseinstellungen das Sekret, d.h. der Mucus überwiegend in Richtung der Lunge in den Patienten hinein transportiert. Diese Art und Weise der Beatmung bewirkt somit indirekt, dass es möglich ist, dass Sekretabsaugungen am Patienten öfters, aufwändiger oder mit längerer zeitlicher Dauer vorgenommen werden müssen. Überwiegt hingegen der exspiratorische Impuls ($S_i$ < 1.0), so wird durch die Art und Weise der Beatmung und die dafür ursächlichen Beatmungseinstellungen ein Transport des Sekretes aus Richtung der Lunge in den Patienten hinaus unterstützt, so dass es vorstellbar ist, dass in der Folge die Notwendigkeit der Häufigkeit, der Aufwand und die Zeitdauer von Sekretabsaugungen reduziert werden kann. Für einen liegenden Patienten kann in idealisierter Weise angenommen werden, dass während einer zeitlichen Phase einer Inspiration (Inspirationsphase) und auch während einer zeitlichen Phase einer Exspiration (Exspirationsphase) die Schwerkraft einen zu vernachlässigenden Einfluss auf die Verlagerung des Sekrets im Bronchialraum eines Patienten hat. Für einen liegenden Patienten stellt damit ein Zielwert von $S_i$ -1.0 einen Zustand dar, in welchem der exspiratorische Impuls und der inspiratorische Impuls in einem Gleichgewicht sind und zugleich ergibt sich daraus die Folge, dass bei diesem Wert von $S_i$ -1.0 kein Sekret in Richtung der Lunge eines Patienten verlagert wird oder sich verlagert. Bei einem sitzenden Patienten verstärkt die Schwerkraft die Verlagerung von Sekret in die Lunge eines Patienten hinein, somit ist ein Zielwert von $S_i$ <1.0, beispielsweise $S_i$ ~ 0.9, anzustreben, um sicherzustellen, dass die Schwerkraft- bedingte Verlagerung und beatmungsbedingte Verlagerung von Sekret in die Lunge eines Patienten in Summe ein Gleichgewicht von inspiratorischen und exspiratorischen Impuls ergeben, so dass im Resultat wiederum kein Sekret in Richtung der Lunge eines Patienten verlagert wird oder sich verlagert.

In einer bevorzugten Ausführung der Medizintechnischen Messeinrichtung sind Mittel zur Ausgabe und/ oder Weiterleitung in oder an der Medizintechnischen Messeinrichtung angeordnet oder mit der medizintechnischen Messeinrichtung verbunden. Die Mittel zur Ausgabe und/ oder Weiterleitung sind ausgebildet, Daten, Zustands- oder Statusinformationen auszugeben oder weiterzuleiten.

Die Mittel zur Ausgabe und/oder Weiterleitung sind vorzugsweise mit bevorzugt vorhandenen Mitteln zur Eingabe in einer Einheit zur Eingabe, Ausgabe und Weiterleitung gemeinsam ausgeführt. Die Einheit zur Eingabe, Ausgabe und Weiterleitung ist bevorzugt ausgebildet, Daten, Zustands- oder Statusinformationen auszugeben oder weiterzuleiten und ist für Eingaben und Bedienung durch einen Anwender vorgesehen.

Dabei ist unter einer Ausgabe, Weiterleitung oder Weitergabe jegliche Form einer Ausgabe, Weiterleitung oder Weitergabe von Informationen, Messwerten, Daten, Zustands- oder Statusinformationen, insbesondere auch die Ausgabe, Weiterleitung oder Weitergabe des erfindungsgemäßen Maßes für die beatmungsbedingte Verlagerung von Sekret zu verstehen. Unter einer Eingabe oder Bedienung ist im Sinne der vorliegenden Erfindung jegliche Aktion oder Interaktion, wie Daten oder Werteingaben, Auslösung und Beendigung von Abläufen, Prozessen oder Prozessschritten an der Medizintechnischen Messvorrichtung durch den Anwender zu verstehen.

Unter einer Ausgabe ist im Sinne der vorliegenden Erfindung jegliche Art und Weise einer optischen, numerischen, visuellen, grafischen, bildlichen, akustischen Ausgabe oder Darstellung zu verstehen.

Unter einer Weitergabe und Weiterleitung von Daten ist im Sinne der vorliegenden Erfindung jegliche Art und Weise einer drahtgebundenen oder drahtlosen Weitergabe und Weiterleitung an ein weiteres Gerät, System, Computersystem, Datennetzwerk, Datenverarbeitungssystem oder Ausgabe- oder Anzeigesystem zu verstehen.

In weiter bevorzugter Weise können die Mittel zur Messwerterfassung, Signalverarbeitung und Berechnung mit der Einheit zur Eingabe, Ausgabe und Weiterleitung als ein gemeinsames Betriebs- und Steuerungsmodul ausgebildet sein. Das Betriebs- und Steuerungsmodul kann teilweise oder insgesamt auch als ein Computerprogramm oder als ein Computerprogrammprodukt bereitgestellt werden, so dass sich der Schutzumfang der vorliegenden Anmeldung ebenfalls auf das Computerprogrammprodukt und das Computerprogramm erstreckt.

In einer weiter bevorzugten Ausführungsform sind die Mittel zur Ausgabe und/ oder Weiterleitung, die Einheit zur Eingabe, Ausgabe und Weiterleitung oder das Betriebs- und Steuerungsmodul ausgebildet, das Maß ($S_i$) für eine beatmungsbedingte Verlagerung von Sekret, ein Ergebnis des Vergleichs zwischen dem Maß für die beatmungsbedingte Verlagerung von Sekret mit einem vorbestimmten Wert oder einen Hinweis bezüglich einer Relation des Maßes der beatmungsbedingten Verlagerung zum vorbestimmten Wert, auszugeben, anzuzeigen oder weiterzuleiten. Der Hinweis und die Art und Weise des Hinweises bezüglich einer Relation des Maßes der beatmungsbedingten Verlagerung ergibt sich in bevorzugter Weise aus einem direkten mathematischen Vergleich des Sekret-Index-Wert ($S_i$) mit dem vorbestimmten Wert oder Schwellwert ($S_{Basis}$). Der Hinweis kann dabei im Sinne der vorliegenden Erfindung auf jegliche Art und Weise einer optischen, numerischen, visuellen, grafischen, bildlichen, akustischen Ausgabe umgesetzt werden. So kann beispielsweise eine Art eines Hinweises einer grafischen Ausgabe in einer Form umgesetzt sein, dass ein Anzeigeelement, beispielsweise ein optisches aktives Element, ein Bildschirmelement, Leuchtmittel oder eine LED, Licht in einer grünen Farbgebung für den Fall abstrahlt, dass der exspiratorische Impuls ($S_i < 0{,}9$) überwiegt, Licht in einer roten Farbgebung für den Fall abstrahlt, dass der inspiratorische Impuls ($S_i > 1.1$) überwiegt, Licht in einer orangen Farbgebung für den Fall abstrahlt, dass der exspiratorische Impuls und der inspiratorische Impuls ($S_i \sim 1.0$) im Bereich der Hysterese ($0{,}9 < S_i < 1{,}1$) nahezu im Gleichgewicht sind.

Die Mittel zur Messwerterfassung, Signalverarbeitung und Berechnung sind bevorzugt ausgebildet, das Maß für eine beatmungsbedingte Verlagerung von Sekret aus einem Quotienten aus der inspiratorischen Messgröße und der exspiratorischen Messgröße zu bestimmen. Die Bildung eines Quotienten aus der inspiratorischen Messgröße und der exspiratorischen Messgröße ergibt in einer vorteilhaften Weise, dass an Hand eines einzelnen Maßes eine Einschätzung möglich ist, in welcher Weise sich die aktuell durchgeführte Beatmung im Hinblick auf die beatmungsbedingte Verlagerung von Sekret auswirkt.

In bevorzugter Weise wird die Sensorik zur Erfassung der inspiratorischen Messgröße durch einen inspiratorischen Durchflusssensor umgesetzt, welcher ausgebildet ist, einen inspiratorischen Betrag einer durch diesen inspiratorischen Durchflusssensor inspiratorisch strömenden Gasmenge quantitativ zu erfassen. In bevorzugter Weise wird die Sensorik zur Erfassung der exspiratorischen Messgröße durch einen exspiratorischen Durchflusssensor umgesetzt, welcher ausgebildet ist, einen exspiratorischen Betrag einer durch diesen exspiratorischen Durchflusssensor exspiratorisch strömenden Gasmenge quantitativ zu erfassen. In einer weiter bevorzugten Weise wird die Sensorik zur Erfassung der inspiratorischen und der exspiratorischen Messgröße durch einen in- und exspiratorischen Durchflusssensor umgesetzt, welcher ausgebildet ist, eine Richtung einer durch diesen in- und exspiratorischen Durchflusssensor strömenden Gasmenge qualitativ zu erfassen und einen Betrag der durch diesen in- und exspiratorischen Durchflusssensor strömenden Gasmenge quantitativ zu erfassen. Der inspiratorische Durchflusssensor und/oder der exspiratorische Durchflusssensor oder der in- und exspiratorische Durchflusssensor sind vorzugsweise mittels eines Mundstücks einer Nasalmaske oder eines Verbindungsstücks, einem sogenannten Y- Stück, mit dem Patienten verbunden. Eine in- und exspiratorische Durchflussmessung an einem Messort mit einem in-und exspiratorischer Durchflusssensor ergibt den Vorteil, dass die in- und exspiratorischen Messwerte zu gleichen Strömungszuständen und zu zeitlich gleichen Messzeitpunkten gewonnen werden, so dass nahezu keinerlei Ungenauigkeit durch zeitliche Verschiebung der in- und exspiratorischen Messwerte zueinander gegeben ist. Dies ist für die Qualität und Genauigkeit bei der Bestimmung als Maßes für die beatmungsbedingte Verlagerung von Sekret, unter Bildung des Quotienten aus der inspiratorischen Messgröße und der exspiratorischen Messgröße von großem Vorteil. In weiter bevorzugter Weise ist der in- und exspiratorische Durchflusssensor ein Patientennaher Durchflusssensor. Weiter bevorzugt ist der Patientennahe Durchflusssensor in oder an dem Verbindungsstück zum Patienten, dem sogenannten Y- Stück angeordnet.

Ein Patientennaher in- und exspiratorischer Durchflusssensor am Y- Stück ergibt den Vorteil, dass zwischen dem Strömungszustand direkt nahe am Patienten und dem Messort keine Veränderungen des Strömungszustandes, durch örtlich nach dem Y-Stück zu durchströmende Komponenten, wie beispielsweise Beatmungsschläuche, Bakterienfilter oder Wasserfallen erfolgen. Diese weiteren Komponenten haben üblicherweise jeweils spezifischen Einfluss auf Druck- und /oder- Strömungsverhältnisse, so dass sich in der Folge eine unterschiedliche Beeinflussung der inspiratorischen und der exspiratorischen Messgröße ergeben kann. Falls diese spezifischen Einflüsse nicht messtechnisch vermindert oder beseitigt und/oder bei der Berechnung mit berücksichtigt werden können, kann sich dadurch ein nachteiliger Einfluss bei der Bestimmung des Maßes für die beatmungsbedingte Verlagerung von Sekret ergeben. Dieser nachteilige Einfluss kann durch die Verwendung des Patientennahen Durchflusssensors am Y- Stück vermindert werden. Durchflusssensoren nach dem Stand der Technik, die in Beatmungsgeräten oder Anästhesiegeräten überwiegend zu Einsatz kommen, erfassen den Durchfluss beispielsweise nach dem Wärmetransportverfahren (Hitzdrahtanemometer, Thermopile, Thermistor), dem Druckdifferenzverfahren ($\Delta P$), oder dem Ultraschall-Laufzeit- Verfahren. Die Anordnung der Durchflusssensoren in der Strömung kann dabei zentriert oder mittig im Strömungskanal, am Rand des Strömungskanals erfolgen. Weiterhin kann durch die Anordnung eine integrale Messung (Hitzdraht-Anordnung in Strömungsmitte zur Erfassung der Hauptströmung), oder eine punktuelle Messung (Thermopile- Anordnung am Rand des Strömungskanals zur Erfassung von Randströmungen oder Wandschubspannungen) des Durchflusses erfolgen.

In einer weiter bevorzugten Weise wird die Sensorik zur Erfassung der inspiratorischen Messgröße durch einen inspiratorischen Drucksensor umgesetzt. In einer weiter bevorzugten Weise wird die Sensorik zur Erfassung der exspiratorischen Messgröße durch einen exspiratorischen Drucksensor umgesetzt.

In einer weiter bevorzugten Ausführungsform werden die exspiratorische und die inspiratorische Messgröße in einem Bezug auf eine zeitliche Dimension umgewandelt. Solche Umwandlungen in Bezug auf die zeitliche Dimension sind eine Bildung eines Integrals ($\int f(t)\ dt$) nach der Zeit oder eine Bildung einer Ableitung ($dx/dt$) nach der Zeit. So ist beispielsweise aus den Messgrößen der Durchflusssensoren mittels der Integralbildung eine Bestimmung eines in- oder exspiratorischen Volumens möglich oder aus den Messgrößen der Drucksensoren ist mittels Differentialrechnung eine Bestimmung eines in- oder exspiratorischen Druckgradienten oder Verlauf eines Druckanstiegs möglich.

In bevorzugter Weise sind der in- und der exspiratorische Druckgradient oder das in- oder das exspiratorische Volumen geeignete Messgrößen, welche Maße für die Transporte von Atemgasen in oder aus der Lunge eines Patienten darstellen. In einer bevorzugten Ausführungsform wird mit den Mitteln zur Messwerterfassung, Signalverarbeitung und Berechnung das Maß für eine beatmungsbedingte Verlagerung von Sekret aus einem inspiratorischen Betrag der inspiratorisch strömenden Gasmenge und einem exspiratorischen Betrag der exspiratorisch strömenden Gasmenge berechnet.

In einer weiter bevorzugten Ausführungsform wird mit den Mitteln zur Messwerterfassung, Signalverarbeitung und Berechnung das Maß für eine beatmungsbedingte Verlagerung von Sekret aus einer Bildung eines Quotienten der inspiratorisch strömenden Gasmenge mit der exspiratorisch strömenden Gasmenge berechnet.

In einer weiter bevorzugten Ausführungsform wird mit den Mitteln zur Messwerterfassung, Signalverarbeitung und Berechnung das Maß für eine beatmungsbedingte Verlagerung von Sekret aus einer Bildung eines Quotienten aus dem mittels einer progressiven mathematischen Beziehung umgewandelten inspiratorischen Betrages der inspiratorisch strömenden Gasmenge und aus dem mittels einer progressiven mathematischen Beziehung umgewandelten exspiratorischen Betrages der exspiratorisch strömenden Gasmenge berechnet. Als mathematische Beziehungen zur Umwandlung des exspiratorischen und inspiratorischen Betrages können beispielsweise quadratische Funktionen, Exponentialfunktionen, Potenzfunktionen oder andere Funktionen oder Zuweisungsvorschriften angewendet werden, welche eine progressive Verstärkung des exspiratorischen und inspiratorischen Betrages bewirken.

Die progressiven mathematisch Beziehungen dienen bei der Ermittlung einer Hervorhebung oder Verstärkung eines durch die Beatmung eines Patienten hervorgerufenen Impulses der inspiratorisch strömenden Gasmenge und eines Impulses der exspiratorischen strömenden Gasmenge, mit dem die Verlagerung von Sekret vom Patienten weg und zum Patienten weg bewirkt wird. Die Bildung des Quotienten aus dem Impuls der inspiratorisch strömenden Gasmenge und aus dem Impuls der exspiratorisch strömenden Gasmenge ergibt einen mittleren Gesamtimpuls als Maß für die beatmungsbedingte Verlagerung von Sekret.

Die progressive Verstärkung ergibt den Vorteil, dass die Empfindlichkeit (Sensitivität) des Maßes für eine beatmungsbedingte Verlagerung von Sekret verbessert wird, da damit große Durchflusssignalamplituden in mathematischer Weise hinsichtlich ihrer Wirkung auf den Impuls der Sekretverlagerung nahezu vergleichbar mit den realen Gegebenheiten in Lunge und Bronchialtrakt abgebildet werden können.

In einer weiter bevorzugten Ausführungsform wird mit den Mitteln zur Messwerterfassung, Signalverarbeitung und Berechnung das Maß für eine beatmungsbedingte Verlagerung von Sekret aus einer Bildung eines Quotienten aus einem Quadrat des inspiratorischen Betrages der inspiratorisch strömenden Gasmenge und aus einem Quadrat des exspiratorischen Betrages der exspiratorisch strömenden Gasmenge berechnet. Die Verwendung des Quotienten aus den Quadraten des in- und des exspiratorischen Betrages ermöglicht eine einfache und transparente Rechenvorschrift, deren Resultat mit einfachen Mitteln überprüfbar, verifizierbar und validierbar ist. Dies ergibt den Vorteil, dass die Berechnung ohne spezielle Anforderungen an die Leistungsfähigkeit der Mittel zur Messwerterfassung, Signalverarbeitung und Berechnung möglich ist, somit auch auf einfachen Prozessorsystemen ($\mu$C) lauffähig ist, wie sie bei Sensorik zur Druck- und Durchflussmessung, beispielsweise als ein sogenanntes Embedded System üblich sind. Diese einfache und transparente Rechenvorschrift einer Quadrierung von Signale zeigt auf, dass es ohne aufwändige Methoden der Signalverarbeitung (FFT- Analyse, Wavelet-Analyse, etc.) möglich ist, ein Maß für eine beatmungsbedingte Verlagerung von Sekret und damit ein Maß für eine Qualität von Beatmungseinstellungen zu bestimmen.

In einer weiter bevorzugten Ausführungsform wird mit den Mitteln zur Messwerterfassung, Signalverarbeitung und Berechnung das Maß für eine beatmungsbedingte Verlagerung von Sekret aus einer Bildung eines Quotienten aus einem zeitlichen Integral eines Quadrates der inspiratorisch strömenden Durchflussmenge (inspiratorischer Volumenstrom $\dot{v}_{insp}$) und aus einem zeitlichen Integral eines Quadrates der exspiratorisch strömenden Durchflussmenge (exspiratorischer Volumenstrom $\dot{v}_{exsp}$)berechnet. Bei einer Erfassung und Auswertung von Durchflusssignalen ist im klinisch-medizinischen Bereich die Bestimmung eines Atem- Minuten- Volumens des Patienten, sowie auch eine Bilanzierung von inspiratorischem und von exspiratorischem Volumen üblich.

Die Bestimmung der Volumina erfolgt über zeitliche Integrale der inspiratorisch und exspiratorisch strömenden Durchflussmengen. Die Bestimmung des Maßes für eine beatmungsbedingte Verlagerung von Sekret aus einer Bildung eines Quotienten aus den zeitlichen Integralen der Quadrate der inspiratorisch und exspiratorisch strömenden Durchflussmengen kann ohne aufwändige Methoden der Signalverarbeitung und Messwerterfassung nahezu mit oder in der gleichen Messwerterfassung mit der Bestimmung der in- und exspiratorischen Volumina erfolgen. Somit kann diese Art der Bestimmung des Maßes für eine beatmungsbedingte Verlagerung von Sekret aus einer Bildung eines Quotienten aus den zeitlichen Integralen der Quadrate der inspiratorisch und exspiratorisch strömenden Durchflussmengen ohne großen konstruktiven Aufwand in vorteilhafter Weise in übliche Systeme der Erfassung und Auswertung von Durchflusssignalen und Volumina mit einbezogen werden.

In einer weiter bevorzugten Ausführungsform wird mit den Mitteln zur Messwerterfassung, Signalverarbeitung und Berechnung das Maß für eine beatmungsbedingte Verlagerung von Sekret aus einer Bildung eines Quotienten aus einem zeitlichen Integral eines Quadrates einer mittels eines inspiratorischen Durchflusssensors erfassten inspiratorisch strömenden Durchflussmenge (inspiratorischer Volumenstrom $\dot{v}_{insp}$) und aus einem zeitlichen Integral eines Quadrates einer mittels eines exspiratorischen Durchflusssensors erfassten exspiratorisch strömenden Durchflussmenge (exspiratorischer Volumenstrom $\dot{v}_{exsp}$) berechnet.

In einer weiter bevorzugten Ausführungsform wird mit den Mitteln zur Messwerterfassung, Signalverarbeitung und Berechnung das Maß für eine beatmungsbedingte Verlagerung von Sekret aus einer Bildung eines Quotienten aus mittels eines in- und exspiratorischen Durchflusssensors erfassten zeitlichen Integrals eines Quadrates einer inspiratorisch strömenden Durchflussmenge (inspiratorischer Volumenstrom $\dot{v}_{insp}$) und eines zeitlichen Integrals eines Quadrates einer exspiratorisch strömenden Durchflussmenge (exspiratorischer Volumenstrom $\dot{v}_{exsp}$) berechnet.

Die folgende Formel 1 gibt in einer allgemeinen Form mit Proportionalitätsfaktoren $k_1$, $k_2$, sowie Skalierungsfunktionen $f_1$, $f_2$, einen mathematischen Zusammenhang für die Bestimmung des Maßes für eine beatmungsbedingte Verlagerung von Sekret als Sekret-Index-Wert ($S_i$) (Mucus- Index) auf Basis einer Quotientenbildung aus dem zeitlichen Integral einer inspiratorischen Messgröße $M_{insp.}$ und dem zeitlichen Integral einer exspiratorischen Messgröße $M_{exsp.}$ an.

$$S_i \approx \frac{k_1 \cdot \int [f_1(M_{insp.})]}{k_2 \cdot \int [f_2(M_{exsp.})]} \qquad \text{Formel 1}$$

Die folgende Formel 2 gibt als eine Basisformel mit Verwendung der inspiratorisch und der exspiratorisch als Volumenströme erfassten Durchflussmengen die Bestimmung des Maßes $S_i$ mit einer Quadrierung der inspiratorischen und exspiratorischen Durchflussmengen als Skalierungsfunktionen $f_1$, $f_2$ für eine beatmungsbedingte Verlagerung von Sekret an.

$$S_i \approx \frac{k_1 \cdot \int (\dot{v}_{insp.}^2)}{k_2 \cdot \int (\dot{v}_{exsp.}^2)} \qquad \text{Formel 2}$$

Mit $k_1 = 1$ und $k_2 = 1$ ergibt sich aus Formel 2 die Formel 3 als vereinfachter Zusammenhang zur Bestimmung des Maßes $S_i$ für eine beatmungsbedingte Verlagerung von Sekret.

$$S_i \approx \frac{\int (\dot{v}_{insp.}^2)}{\int (\dot{v}_{exsp.}^2)} \qquad \text{Formel 3}$$

Die Integralbildung erfolgt in den Formeln 1 bis 3 für Integrationsintervalle von mindestens einer Inspirationsphase und von mindestens einer Exspirationsphase, wobei für die inspiratorischen und exspiratorischen Integrationsintervalle eine jeweils gleiche Anzahl von Atemphasen einbezogen wird.

Eine weitere erfindungsgemäße Ausführungsform einer Vorrichtung mit der eine Verlagerung von Sekret in die Lunge eines Patienten möglichst gering gehalten werden kann, stellt eine Beatmungsvorrichtung mit einem Beatmungssystem und mit der erfindungsgemäßen medizintechnischen Messvorrichtung dar.

Ein derartiges Beatmungssystem besteht aus den Komponenten Aktuatorik, Sensorik, einer Kontrolleinheit zum Betrieb des Beatmungssystems und/oder zu einer Steuerung und/oder zu einer Regelung einer Beatmung, Mitteln zur Eingabe, Ausgabe und Weiterleitung zu einer Bedienung des Beatmungssystems oder der Beatmungsvorrichtung durch einen Anwender und/oder zu einem Datenaustausch mit anderen Geräten oder Systemen, Verbindungsmitteln zu einer pneumatischen, gasführende Verbindung des Beatmungssystems oder der Beatmungsvorrichtung mit einem Patienten. Die Mittel zur Eingabe, Ausgabe und Weiterleitung können, neben Elementen für Eingaben oder Bedienungen der medizintechnischen Messeinrichtung im Sinne der vorliegenden Erfindung, auch Mittel zu Eingaben oder Bedienungen, wie Start/Stopp der Beatmung, Mittel zur Auswahl und/oder Aktivierung von Beatmungsformen, Mittel zur Eingabe von Konfigurationen, insbesondere auch zu patientenspezifischen Konfigurationen der Beatmungsvorrichtung, mit umfassen. Besonders bevorzugt sind die Mittel zur Eingabe, Ausgabe und Weiterleitung als ein Bestandteil oder ein Modul der Kontrolleinheit ausgeführt. Weiter bevorzugt können auch Elemente der medizintechnischen Messeinrichtung, wie beispielsweise die Mittel zur Messwerterfassung, Signalverarbeitung und Berechnung, in die Kontrolleinheit eingebunden oder an diese angebunden bzw. gekoppelt sein. Die pneumatische oder gasführende Verbindung mit dem Patienten erfolgt vorzugsweise über ein Beatmungsschlauchsystem, wobei das Beatmungsschlauchsystem als ein sogenanntes Zwei- Schlauchsysteme mit einer inspiratorischen Zuleitung zum Patienten hin und mit einer exspiratorischen Fortleitung vom Patienten weg oder als ein sogenanntes Ein- Schlauchsystem mit lediglich einer inspiratorischen Zuleitung zum Patienten hin ausgeführt sein kann.

Unter dem Sammelbegriff der Aktuatorik sind im Sinne der vorliegenden Erfindung jegliche Komponenten zur Dosierung von Gasen oder Flüssigkeiten, Ventile, wie beispielsweise Dosierventile, insbesondere inspiratorische Dosierventile und Exspirationsventile, Rückschlagventile, Sicherheitsventile, sowie Komponenten zur Erzeugung von Gasdrücken, Gasmengen oder Gasdurchflussmengen, wie beispielsweise Druckquellen oder Beatmungsantriebe, wie beispielsweise Kolbenpumpen, Faltenbalgantriebe, Turbinenantriebe oder Rotations- oder Radialverdichter (Blower), sowie Seitenkanalverdichter zu verstehen.

Unter einer Beatmungsvorrichtung oder einer aus einem Beatmungssystem und einer Medizintechnischen Messvorrichtung fortgebildeten Beatmungsvorrichtung sind im Sinne der vorliegenden Erfindung einerseits Beatmungsgeräte zum Einsatz im klinischen Umfeld in der Notaufnahme, zum Einsatz auf diagnostischen Stationen, zum Einsatz auf Patientenstationen und zum Einsatz während innerklinischer Transporte, Intensiv- Beatmungsgeräte zu einem Einsatz auf einer Intensivstation, Notfall-Beatmungsgeräte zu einem mobilen Einsatz in Notfall- und Transportsituationen und andererseits auch Anästhesiegeräte zur Verwendung unterhalb einer Operation oder im Einleitungs- oder Aufwachraum zu verstehen. In einer bevorzugten Ausführungsform der Beatmungsvorrichtung sind die Einheit zur Messwerterfassung, Signalverarbeitung und Berechnung oder die Kontrolleinheit ausgebildet, das Maß für die beatmungsbedingte Verlagerung von Sekret mit einem vorbestimmten Wert zu vergleichen.

In einer weiteren Ausführungsform der Beatmungsvorrichtung sind die Mittel zur Eingabe, Ausgabe und Weiterleitung ausgebildet, das Maß für die beatmungsbedingte Verlagerung von Sekret, ein Ergebnis des Vergleichs zwischen dem Maß für die beatmungsbedingte Verlagerung von Sekret mit dem vorbestimmten Wert oder einen Hinweis bezüglich einer Relation des Maßes der beatmungsbedingten Verlagerung zum vorbestimmten Wert, weiterzuleiten oder an einen Anwender auszugegeben.

Weiter bevorzugt kann in oder an der Beatmungseinrichtung vorgesehen sein, dass die Kontrolleinheit oder Einheit zur Eingabe, Ausgabe und Weiterleitung ausgebildet sind, einen Hinweis zur Anpassung eines oder mehrerer Beatmungsparameter, einen Hinweis zur gewählten Beatmungsform in Bezug auf die beatmungsbedingte Verlagerung von Sekret oder einen Hinweis zur Anpassung eines oder mehrerer Beatmungsparameter mit mindestens einem Vorschlagswert für den mindestens einen Beatmungsparameter oder die Beatmungsform auszugeben.

In einer Variante dieser weiter bevorzugten Ausbildung ist die Beatmungsvorrichtung mit der Einheit zur Eingabe, Ausgabe und Weiterleitung und der Kontrolleinheit ausgebildet, eine Bestätigung für den mindestens einen Vorschlagswert anzufordern, die Bestätigung als Eingabe aufzunehmen und den Vorschlagswert für einen weiteren Betrieb der Beatmungsvorrichtung zu verwenden.

In einer weiteren Ausführungsform der Beatmungsvorrichtung sind die Kontrolleinheit und die Aktuatorik ausgebildet, bei einer Abweichung des Maßes der beatmungsbedingten Verlagerung von Sekret von einem vorbestimmten Wert, einen Stellwert eines Inspirationsdrucks zu verändern oder anzupassen, einen Stellwert einer Inspirations- Durchflussmenge oder einen Höchstwert einer Inspirations- Durchflussmenge zu verändern oder anzupassen, einen Anstieg einer Druckrampe des Inspirationsdrucks zu verändern oder anzupassen, eine Anstiegszeitdauer der Druckrampe des Inspirationsdrucks zu verändern oder anzupassen, ein Inspirations- zu Exspirationsverhältnis, eine inspiratorische Pause in der Inspirationsphase oder eine Beatmungsfrequenz zu verändern oder anzupassen.

In einer weiter bevorzugten Variante der weiteren Ausführungsform der Beatmungsvorrichtung wird durch die Kontrolleinheit in Zusammenwirken mit der Aktuatorik bei einer Überschreitung des vorbestimmten Wertes durch das Maß der beatmungsbedingten Verlagerung von Sekret der Stellwert des Inspirationsdrucks verringert.

In einer weiter bevorzugten Variante der weiteren Ausführungsform der Beatmungsvorrichtung wird durch die Kontrolleinheit in Zusammenwirken mit der Aktuatorik bei einer Unterschreitung des vorbestimmten Wertes durch das Maß der beatmungsbedingten Verlagerung von Sekret der Stellwert des Inspirationsdrucks bei einer Unterschreitung des vorbestimmten Wertes durch das Maß der beatmungsbedingten Verlagerung von Sekret erhöht.

In einer weiter bevorzugten Variante der weiteren Ausführungsform der Beatmungsvorrichtung wird durch die Kontrolleinheit in Zusammenwirkung mit der Aktuatorik bei einer Überschreitung des vorbestimmten Wertes durch das Maß der beatmungsbedingten Verlagerung von Sekret der Stellwert der Inspirations-Durchflussmenge verringert. In einer weiter bevorzugten Variante der weiteren Ausführungsform der Beatmungsvorrichtung wird durch die Kontrolleinheit in Zusammenwirkung mit der Aktuatorik bei einer Unterschreitung des vorbestimmten Wertes durch das Maß der beatmungsbedingten Verlagerung von Sekret der Stellwert der Inspirations- Durchflussmenge erhöht.

In einer weiter bevorzugten Variante der weiteren Ausführungsform der Beatmungsvorrichtung wird durch die Kontrolleinheit in Zusammenwirkung mit der Aktuatorik bei einer Überschreitung des vorbestimmten Wertes durch das Maß der beatmungsbedingten Verlagerung von Sekret der Höchstwert der Inspirations-Durchflussmenge begrenzt. In einer weiter bevorzugten Variante der weiteren Ausführungsform der Beatmungsvorrichtung wird durch die Kontrolleinheit in Zusammenwirkung mit der Aktuatorik bei einer Unterschreitung des vorbestimmten Wertes durch das Maß der beatmungsbedingten Verlagerung von Sekret die Begrenzung des Höchstwertes der Inspirations- Durchflussmenge aufgehoben.

Die Begrenzung des Höchstwertes der Inspirations- Durchflussmenge, sowie die Aufhebung der Begrenzung des Höchstwertes der Inspirations- Durchflussmenge wird in einer besonderen Ausführungsform stufenweise vorgenommen.

In einer weiteren Ausführungsform wird die Begrenzung des Höchstwertes der Inspirations- Durchflussmenge in Bezug zum Höchstwert der Exspirations-Durchflussmenge vorgenommen. Die Formel 4 zeigt die Beziehung zwischen den Höchstwerten der Inspirations- Durchflussmenge ($\dot{v}_{max\_insp.}$) und der Exspirations- Durchflussmenge ($\dot{v}_{max\_exsp.}$) auf

$$\dot{v}_{max\_insp.} \approx k_3 \cdot \dot{v}_{max\_exsp.} \qquad \text{Formel 4}$$

Dabei wird k3 vorzugsweise in einem Bereich von 0,45 bis 0,95 gewählt, so dass sichergestellt ist, dass der Höchstwert der Exspirations- Durchflussmenge grösser als der Höchstwert der Inspirations- Durchflussmenge ist. Die Vorteile und die Wirkung einer Begrenzung des Höchstwertes der Inspirations- Durchflussmenge sind aus der Formel 3 ersichtlich. Der Sekret-Index-Wert ($S_i$) (Mucus- Index) wird reduziert, indem inspiratorische Durchflussmengen mit großem Anteil am Impuls der Verlagerung von Sekret reduziert werden.

In einer weiter bevorzugten Variante der weiteren Ausführungsform der Beatmungsvorrichtung wird durch die Kontrolleinheit in Zusammenwirkung mit der Aktuatorik bei einer Überschreitung des vorbestimmten Wertes durch das Maß der beatmungsbedingten Verlagerung von Sekret der Anstieg der Druckrampe des Inspirationsdrucks verringert. In einer weiter bevorzugten Variante der weiteren Ausführungsform der Beatmungsvorrichtung wird durch die Kontrolleinheit in Zusammenwirkung mit der Aktuatorik bei einer Unterschreitung des vorbestimmten Wertes durch das Maß der beatmungsbedingten Verlagerung von Sekret der Anstieg der Druckrampe des Inspirationsdrucks erhöht.

In einer weiter bevorzugten Variante der weiteren Ausführungsform der Beatmungsvorrichtung wird durch die Kontrolleinheit in Zusammenwirkung mit der Aktuatorik bei einer Überschreitung des vorbestimmten Wertes durch das Maß der beatmungsbedingten Verlagerung von Sekret die Anstiegszeitdauer der Druckrampe des verlängert. In einer weiter bevorzugten Variante der weiteren Ausführungsform der Beatmungsvorrichtung wird durch die Kontrolleinheit in Zusammenwirkung mit der Aktuatorik bei einer Unterschreitung des vorbestimmten Wertes durch das Maß der beatmungsbedingten Verlagerung von Sekret die Anstiegszeitdauer der Druckrampe des Inspirationsdrucks verkürzt.

In einer weiter bevorzugten Variante der weiteren Ausführungsform der Beatmungsvorrichtung wird durch die Kontrolleinheit in Zusammenwirkung mit der Aktuatorik bei einer Überschreitung des vorbestimmten Wertes durch das Maß der beatmungsbedingten Verlagerung von Sekret in der Inspirationsphase eine inspiratorische Pause verlängert. In einer weiter bevorzugten Variante der weiteren Ausführungsform der Beatmungsvorrichtung wird durch die Kontrolleiriheit in Zusammenwirkung mit der Aktuatorik bei einer Unterschreitung des vorbestimmten Wertes durch das Maß der beatmungsbedingten Verlagerung von Sekret in der Inspirationsphase die inspiratorische Pause die inspiratorische Pause verkürzt.

In einer weiteren Ausführungsform der Beatmungsvorrichtung sind die Kontrolleinheit und die Aktuatorik zusammenwirkend ausgebildet, bei einer Überschreitung des vorbestimmten Wertes durch das Maß der beatmungsbedingten Verlagerung von Sekret, einen Stellwert eines Inspirationsdrucks zu verringern, einen Anstieg einer Druckrampe des Inspirationsdrucks zu verringern oder eine Anstiegszeitdauer der Druckrampe des Inspirationsdrucks zu verlängern, so dass das Maß der beatmungsbedingten Verlagerung von Sekret den vorbestimmten Wert in der Folge wieder unterschreitet.

In dieser weiteren Ausführungsform der Beatmungsvorrichtung sind die Kontrolleinheit und die Aktuatorik bevorzugt weiterhin zusammenwirkend ausgebildet, bei einer Unterschreitung des vorbestimmten Wertes durch das Maß der beatmungsbedingten Verlagerung von Sekret, den Stellwert des Inspirationsdrucks zu erhöhen, den Anstieg der Druckrampe des Inspirationsdrucks zu erhöhen oder die Anstiegszeitdauer der Druckrampe des Inspirationsdrucks zu verkürzen, so dass das Maß der beatmungsbedingten Verlagerung von Sekret den vorbestimmten Wert in der Folge wieder überschreitet.

In einer weiteren Ausführungsform der Beatmungsvorrichtung sind die Kontrolleinheit und die Aktuatorik zusammenwirkend ausgebildet, bei einer Überschreitung des vorbestimmten Wertes durch das Maß der beatmungsbedingten Verlagerung von Sekret, ein Inspirations- zu Exspirationsverhältnis und/oder eine Beatmungsfrequenz und/oder eine inspiratorische Pause in der Inspirationsphase zu verändern oder anzupassen, so dass das Maß der beatmungsbedingten Verlagerung von Sekret den vorbestimmten Wert in der Folge wieder unterschreitet.

In dieser weiteren Ausführungsform der Beatmungsvorrichtung sind die Kontrolleinheit und die Aktuatorik bevorzugt weiterhin zusammenwirkend ausgebildet, bei einer Unterschreitung des vorbestimmten Wertes durch das Maß der beatmungsbedingten Verlagerung von Sekret, das Inspirations- zu Exspirationsverhältnis und/oder die Beatmungsfrequenz und/oder die inspiratorische Pause in der Inspirationsphase zu verändern oder anzupassen, so dass das Maß der beatmungsbedingten Verlagerung von Sekret den vorbestimmten Wert in der Folge wieder überschreitet.

In dieser weiteren Ausführungsform der Beatmungsvorrichtung sind die Kontrolleinheit und die Aktuatorik bevorzugt weiterhin zusammenwirkend ausgebildet, bei einer Unterschreitung des vorbestimmten Wertes durch das Maß der beatmungsbedingten Verlagerung von Sekret, in der Inspirationsphase eine inspiratorische Pause zu verlängern, so dass das Maß der beatmungsbedingten Verlagerung von Sekret den vorbestimmten Wert in der Folge wieder überschreitet.

In einer weiteren Ausführungsform der Beatmungsvorrichtung wird durch die Kontrolleinheit in Zusammenwirkung mit der Aktuatorik bei einer Überschreitung des vorbestimmten Wertes durch das Maß der beatmungsbedingten Verlagerung von Sekret der Höchstwert der Inspirations- Durchflussmenge schrittweise begrenzt, bis das Maß der beatmungsbedingten Verlagerung von Sekret den vorbestimmten Wert wieder unterschreitet.

In dieser weiteren Ausführungsform der Beatmungsvorrichtung wird bevorzugt durch die Kontrolleinheit in Zusammenwirkung mit der Aktuatorik bei einer Unterschreitung des vorbestimmten Wertes durch das Maß der beatmungsbedingten Verlagerung von Sekret die Begrenzung des Höchstwertes der Inspirations- Durchflussmenge schrittweise aufgehoben, bis das Maß der beatmungsbedingten Verlagerung von Sekret den vorbestimmten Wert wieder überschreitet.

Ein erfindungsgemäßes Verfahren zum Betrieb einer Medizinischen Messvorrichtung oder ein Verfahren zum Betrieb einer Beatmungsvorrichtung mit einer verringerten Verlagerung von Sekret in die Lunge sind durch eine Schrittabfolge mit mehreren Schritten oder Teilschritten ausgebildet.

Zu einer Durchführung des erfindungsgemäßen Verfahrens mit einer verringerten Verlagerung von Sekret in die Lunge ist die Beatmungsvorrichtung bevorzugt als Beatmungs- und Anästhesiegerät ausgebildet, wobei zur Steuerung und zum Betrieb des Beatmungs- und Anästhesiegerätes vorzugsweise in dem Beatmungs-und Anästhesiegerät mindestens ein Betriebs- und Steuerungsmodul vorgesehen ist.

Weiter bevorzugt wird die Abfolge von Schritten durch mindestens ein Betriebs-und Steuerungsmodul ausgeführt. Das Betriebs- und Steuerungsmodul kann teilweise oder insgesamt auch als ein Computerprogramm oder als ein Computerprogrammprodukt bereitgestellt werden.

Mithin erkennt man, dass sich der Schutzumfang der vorliegenden Anmeldung ebenfalls auf das Computerprogrammprodukt und das Computerprogramm erstrecken kann.

Das Betriebs- und Steuerungsmodul umfasst vorzugsweise die Mittel zur Messwerterfassung, Signalverarbeitung und Berechnung, die Kontrolleinheit, sowie bevorzugt Mittel zur Datenspeicherung und die Mittel zur Eingabe, Ausgabe und Weiterleitung.

In dem erfindungsgemäßen Verfahren zum Betrieb einer Medizintechnischen Messvorrichtung oder zum Betrieb einer Beatmungsvorrichtung werden in einem ersten Schritt mittels der Sensorik und mit den Mitteln zur Messwerterfassung, Signalverarbeitung und Berechnung eine inspiratorische Messgröße und eine exspiratorische Messgröße erfasst,

in einem zweiten Schritt wird mit den Mitteln zur Messwerterfassung, Signalverarbeitung und Berechnung die inspiratorische Messgröße mittels einer ersten progressiven mathematischen Beziehung in eine inspiratorische Impulskenngröße und die exspiratorische Messgröße mittels einer zweiten progressiven mathematischen Beziehung in eine exspiratorische Impulskenngröße mathematisch umgewandelt,

in einem dritten Schritt wird mit den Mitteln zur Messwerterfassung, Signalverarbeitung und Berechnung ein Quotient aus der inspiratorischen Impulskenngröße und der exspiratorischen Impulskenngröße als ein Maß für eine beatmungsbedingte Verlagerung von Sekret gebildet.

In einer bevorzugten Ausführungsform des Verfahrens wird in einem weiteren Schritt mit den Mitteln zur Eingabe, Ausgabe und Weiterleitung das Maß für die beatmungsbedingte Verlagerung von Sekret ausgegeben oder weitergeleitet.

In einer weiter bevorzugten Ausführungsform des Verfahrens wird in einem weiteren Schritt mit den Mitteln zur Messwerterfassung, Signalverarbeitung und Berechnung das Maß für die beatmungsbedingte Verlagerung von Sekret mit einem vorbestimmten Wert verglichen und mittels der Mittel zur Eingabe, Ausgabe und Weiterleitung ein Hinweis bezüglich einer Relation des Maßes der beatmungsbedingten Verlagerung zum vorbestimmten Wert weitergeleitet oder an einen Anwender ausgegeben. In einer besonderen Variante dieser weiter bevorzugten Ausführungsform wird in diesem weiteren Schritt mit den Mitteln zur Eingabe, Ausgabe und Weiterleitung ein Hinweis zur Anpassung eines oder mehrerer Beatmungsparameter, ein Hinweis zur gewählten Beatmungsform in Bezug auf die beatmungsbedingte Verlagerung von Sekret oder ein Hinweis zur Anpassung eines oder mehrerer Beatmungsparameter mit mindestens einem Vorschlagswert für den mindestens einen Beatmungsparameter oder die Beatmungsform ausgegeben.

In einem folgenden weiteren Schritt wird in dieser besonderen Variante dieser weiter bevorzugten Ausführungsform mittels der Kontrolleinheit in Verbindung mit den Mitteln zur Eingabe, Ausgabe und Weiterleitung eine Bestätigung für den mindestens einen Vorschlagswert angefordert und in einem darauf folgenden weiteren Schritt wird nach Eingang der Bestätigung an der Einheit zur Eingabe, Ausgabe und Weiterleitung dieser Vorschlagswert von der Kontrolleinheit für einen weiteren Betrieb der Beatmungsvorrichtung verwendet.

In einer weiteren Ausführungsform des Verfahrens wird in einem weiteren Schritt mit den Mitteln zur Messwerterfassung, Signalverarbeitung und Berechnung das Maß für die beatmungsbedingte Verlagerung von Sekret mit einem vorbestimmten Wert verglichen und bei einer Abweichung des Maßes für die beatmungsbedingte Verlagerung von Sekret vom vorbestimmten Wert wird mittels der Kontrolleinheit und der Aktuatorik ein Inspirationsdruck verändert oder angepasst wird, ein Stellwert oder ein Höchstwert eines Inspirations- Durchflusses verändert oder angepasst, ein Anstieg einer Druckrampe eines Inspirationsdrucks verändert oder angepasst oder eine Anstiegszeitdauer der Druckrampe des Inspirationsdrucks verändert oder angepasst.

In einer weiter bevorzugten Variante der weiteren Ausführungsform des Verfahrens wird in einem weiteren Schritt der Stellwert des Inspirationsdrucks bei einer Überschreitung des vorbestimmten Wertes durch das Maß der beatmungsbedingten Verlagerung von Sekret verringert.

In einer weiter bevorzugten Variante der weiteren Ausführungsform des Verfahrens wird bei einer Unterschreitung des vorbestimmten Wertes durch das Maß der beatmungsbedingten Verlagerung von Sekret in einem weiteren Schritt der Stellwert des Inspirationsdrucks der Stellwert des Inspirationsdrucks erhöht.

In einer weiter bevorzugten Variante der weiteren Ausführungsform des Verfahrens wird bei einer Überschreitung des vorbestimmten Wertes durch das Maß der beatmungsbedingten Verlagerung von Sekret in einem weiteren Schritt der Anstieg der Druckrampe des Inspirationsdrucks verringert.

In einer weiter bevorzugten Variante der weiteren Ausführungsform des Verfahrens wird bei einer Unterschreitung des vorbestimmten Wertes durch das Maß der beatmungsbedingten Verlagerung der Anstieg der Druckrampe des Inspirationsdrucks erhöht.

In einer weiter bevorzugten Variante der weiteren Ausführungsform des Verfahrens wird bei einer Überschreitung des vorbestimmten Wertes durch das Maß der beatmungsbedingten Verlagerung von Sekret in einem weiteren Schritt die Anstiegszeitdauer der Druckrampe des Inspirationsdrucks verlängert.

In einer weiter bevorzugten Variante der weiteren Ausführungsform des Verfahrens wird bei einer Unterschreitung des vorbestimmten Wertes durch das Maß der beatmungsbedingten Verlagerung von Sekret die Anstiegszeitdauer der Druckrampe des Inspirationsdrucks verkürzt.

In einer weiteren bevorzugten Ausführungsform des Verfahrens wird in einem weiteren Schritt mit den Mitteln zur Messwerterfassung, Signalverarbeitung und Berechnung das Maß für die beatmungsbedingte Verlagerung von Sekret mit einem vorbestimmten Wert verglichen und bei einer Überschreitung des vorbestimmten Wertes wird mittels der Kontrolleinheit und der Aktuatorik ein Stellwert eines Inspirationsdrucks verringert, ein Anstieg einer Druckrampe des Inspirationsdrucks verringert, ein Stellwert eines Inspirations- Durchflusses verringert, ein Höchstwert des Inspirations- Durchflusses begrenzt oder eine Anstiegszeitdauer der Druckrampe des Inspirationsdrucks verlängert, so dass das Maß der beatmungsbedingten Verlagerung von Sekret den vorbestimmten Wert in der Folge wieder unterschreitet.

In einer weiteren bevorzugten Ausführungsform des Verfahrens wird in einem weiteren Schritt mit den Mitteln zur Messwerterfassung, Signalverarbeitung und Berechnung das Maß für die beatmungsbedingte Verlagerung von Sekret mit einem vorbestimmten Wert verglichen und bei einer Überschreitung des vorbestimmten Wertes wird mittels der Kontrolleinheit und der Aktuatorik ein Inspirations- zu Exspirationsverhältnis und/oder eine Beatmungsfrequenz derart verändert, so dass das Maß der beatmungsbedingten Verlagerung von Sekret den vorbestimmten Wert in der Folge wieder unterschreitet.

Die vorstehend beschriebenen, erfindungsgemäßen Ausführungsformen des Verfahrens können auch als ein Computerprogrammprodukt mit einem Computerprogramm ausgebildet sein, wobei ein Computer zur Durchführung des oben beschriebenen, erfindungsgemäßen Verfahrens veranlasst wird, wenn das Computerprogramm auf dem Computer bzw. auf einem Prozessor des Computers ausgeführt wird. Eine alternative Aufgabenlösung besteht auch in einem Computerprogramm mit Computer-Programmcode zur Durchführung aller Verfahrensschritte des beanspruchten oder oben beschriebenen Verfahrens, wenn das Computerprogramm auf dem Computer ausgeführt wird. Dabei kann das Computerprogramm auch auf einem maschinenlesbaren Speichermedium gespeichert sein. Eine alternative Aufgabenlösung sieht ein Speichermedium vor, das zur Speicherung des vorstehend beschriebenen, computer-implementierten Verfahrens bestimmt ist und von einem Computer lesbar ist.

Es liegt im Rahmen der Erfindung, dass nicht alle Schritte des Verfahrens zwangsläufig auf ein und derselben Computerinstanz ausgeführt werden müssen, sondern sie können auch auf unterschiedlichen Computerinstanzen ausgeführt werden. Auch kann die Abfolge der Verfahrensschritte gegebenenfalls variiert werden. Darüber hinaus ist es möglich, dass einzelne Abschnitte des vorstehend beschriebenen Verfahrens in einer verkaufsfähigen Einheit (z. B. der Einheit zur Eingabe-Ausgabe-Weiterleitung) und die restlichen Komponenten in einer anderen verkaufsfähigen Einheit (z.B. der medizintechnischen Messvorrichtung, dem Betriebs- und Steuerungsmodul oder dem Gerät an sich) - sozusagen als verteiltes System - ausgeführt werden können.

Vorstehend wurde die Lösung der Aufgabe in Bezug auf das beanspruchte Verfahren beschrieben. Hierbei erwähnte Merkmale, Vorteile oder alternative Ausführungsformen sind ebenso auch auf die anderen beanspruchten Gegenstände der medizintechnischen Messvorrichtung oder der Beatmungsvorrichtung zu übertragen und umgekehrt. Mit anderen Worten können die gegenständlichen Ansprüche (die beispielsweise auf ein Modul oder auf ein Gerät gerichtet sind) auch mit den Merkmalen, die in Zusammenhang mit dem Verfahren beschrieben oder beansprucht sind, weitergebildet sein. Die entsprechenden funktionalen Merkmale des Verfahrens werden dabei durch entsprechende gegenständliche Module, insbesondere durch Hardware-Bausteine, ausgebildet, die beispielsweise in Form eines Mikroprozessors ($\mu$P), Mikrocontrollers ($\mu$C) oder in Form von Instruktionen implementiert sein können, die in einem elektronischen Schaltkreis in einem Speicherbaustein abgelegt sind und durch einen Prozessor verarbeitet werden.

Eine erfindungsgemäße Verwendung der Medizintechnischen Messvorrichtung wird dadurch ausgebildet, dass mit den Mitteln zur Eingabe, Ausgabe und Weiterleitung eine Bestätigung für den mindestens einen Vorschlagswert angefordert wird, ein Bestätigungs- spezifisches Signal erfasst wird, das Bestätigungs- spezifische Signal zu einem Steuersignal verarbeitet wird und das Steuersignal an eine Kontrolleinheit einer Beatmungsvorrichtung bereitgestellt wird.

Die vorliegende Erfindung wird anhand von Figuren im Folgenden näher erläutert. Es zeigen:

Fig. 1    eine Medizintechnische Messvorrichtung,

Fig. 2    eine Beatmungsvorrichtung,

Fig. 3    einen schematischen Ablaufplan eines Verfahrens zum Betrieb einer Medizintechnischen Messvorrichtung.

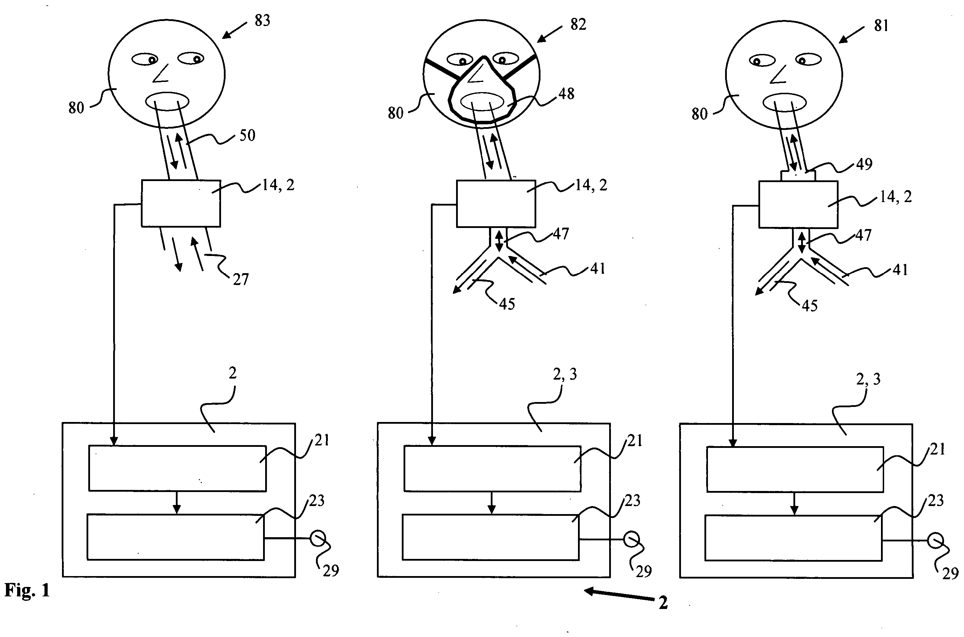

In der Figur 1 ist eine medizintechnische Messvorrichtung 2 in Verbindung mit einem Patienten 80 gezeigt, welche in drei Darstellungsvarianten 81, 82, 83 drei unterschiedliche Verbindungsmöglichkeiten der medizintechnischen Messvorrichtung 2 mit dem Patienten 80 veranschaulicht. Der Patient 80 wird in der ersten Darstellungsvariante 81 über einen Endotrachialtubus 49 mit Atemgas versorgt. In der zweiten Darstellungsvariante 82 wird der Patient 80 über eine Nasalmaske 48 mit Atemgas versorgt. Sowohl die Nasalmaske 48, als auch der Endotrachialtubus 49 sind pneumatisch mit einem in- und exspiratorischen Durchflusssensor 14 verbunden. An den in- und exspiratorischen Durchflusssensor 14 schließt sich mittels eines Verbindungsstückes 47, eines sogenannten Y-Stückes 47, eines exspiratorischen Beatmungsschlauches 45 und eines inspiratorischen Beatmungsschlauches 41 eine Verbindung mit einem - in dieser Figur 1 nicht dargestellten - Beatmungsgerät, Neonatal-Beatmungsgerät, Notfall-Beatmungsgerät oder Anästhesiegerät an. In einer besonderen Variante kann der in- und exspiratorische Durchflusssensor 14 konstruktiv in oder an das Verbindungstück 47 gekoppelt oder in dieses Verbindungstück 47 integriert sein.

Die Verbindung eines Patienten 80 zu einem Beatmungsgerät ist zu einer Beatmung eines Patienten 80 vorgesehen und erforderlich. In der dritten Darstellungsvariante 83 ist der Patient 80 über ein Mundstück 50 mit der medizintechnischen Messvorrichtung 2 gasführend verbunden, ohne dass eine Verbindung zu dem Beatmungsgerät gegeben ist. Der Patient atmet durch das Mundstück 50 und den in- und exspiratorischen Durchflusssensor 14 Atemluft frei aus einer Umgebung 27 ein und frei in die Umgebung 27 wieder aus.

Die medizintechnische Messvorrichtung 2 weist eine Einheit zur Messwerterfassung, Signalverarbeitung und Berechnung 21 mit Mitteln zur Messwerterfassung, Mitteln zur Signalverarbeitung und Mitteln zur Berechnung und eine Einheit zur Eingabe, Ausgabe und Weiterleitung 23 mit Mitteln zur Eingabe, wie beispielsweise Tast- und Schaltelemente, berührungssensitive Bildschirme Tastaturen, Mitteln zur Ausgabe, wie beispielsweise Leuchtmittel, optische und/ oder akustische Signalmittel, Bildschirme, und Mitteln zur Weiterleitung, wie beispielsweise drahtgebundene oder drahtlose Schnittstellen, auf. Die Einheit zur Messwerterfassung, Signalverarbeitung und Berechnung 21 und die Einheit zur Eingabe, Ausgabe und Weiterleitung 23 sind in dieser Ausführung nach Figur 1 gemeinsam als ein zentrales Betriebs- und Steuerungsmodul 3 ausgebildet. Somit bilden in dieser Figur 1 der in- und exspiratorische Durchflusssensor 14 und das zentrale Betriebs- und Steuerungsmodul 3 zusammen im Wesentlichen die medizintechnische Messvorrichtung 2.

An der Einheit zur Eingabe, Ausgabe und Weiterleitung 23 ist eine Schnittstelle 29 zum Austausch von Daten mit - in dieser Figur 1 nicht gezeigten - externen Gerätschaften (Bildschirme, Beatmungsgeräte, Überwachungssysteme) oder Netzwerken vorgesehen. Diese Schnittstelle 29 kann drahtgebunden, beispielsweise als Datennetzwerk, LAN, Ethernet, VGA, DVI, HDMI, USB, RS232, RS485, TOS-Link oder drahtlos, beispielsweise als Funknetzwerk, WLAN, IrDA, IrOBEX ausgeführt sein. Der in- und exspiratorische Durchflusssensor 14 ist mit der Einheit zur Messwerterfassung, Signalverarbeitung und Berechnung 21 daten- und signaltechnisch verbunden, in der Einheit zur Messwerterfassung, Signalverarbeitung und Berechnung 21 werden eine, mit dem in- und exspiratorische Durchflusssensor 14 erfasste, inspiratorische Durchflussmenge und eine exspiratorische Durchflussmenge mittels einer der Formeln 2 oder 3 basierenden Berechnung in das Maß $S_i$ für eine beatmungsbedingte Verlagerung von Sekret umgerechnet. Dieses Maß $S_i$ wird von der Einheit zur Messwerterfassung, Signalverarbeitung und Berechnung 21 an die Einheit zur Eingabe, Ausgabe und Weiterleitung 23 weitergeleitet, wobei in der Einheit zur Messwerterfassung, Signalverarbeitung und Berechnung 21 auch eine Einordnung des Maßes in einen vorbestimmten Werteraum, z.B. mit Hilfe eines Schwellwertvergleiches vorgenommen werden kann. Mit Hilfe der Einheit zur Eingabe, Ausgabe und Weiterleitung 23 wird das Maß $S_i$ direkt, beispielsweise numerisch, grafisch oder in aufbereiteter Form, beispielsweise in Relation zu einem Schwellwert in der Farbgebung adaptiert an den Anwender ausgegeben und/ oder an der Schnittstelle 29 zur Verfügung gestellt.

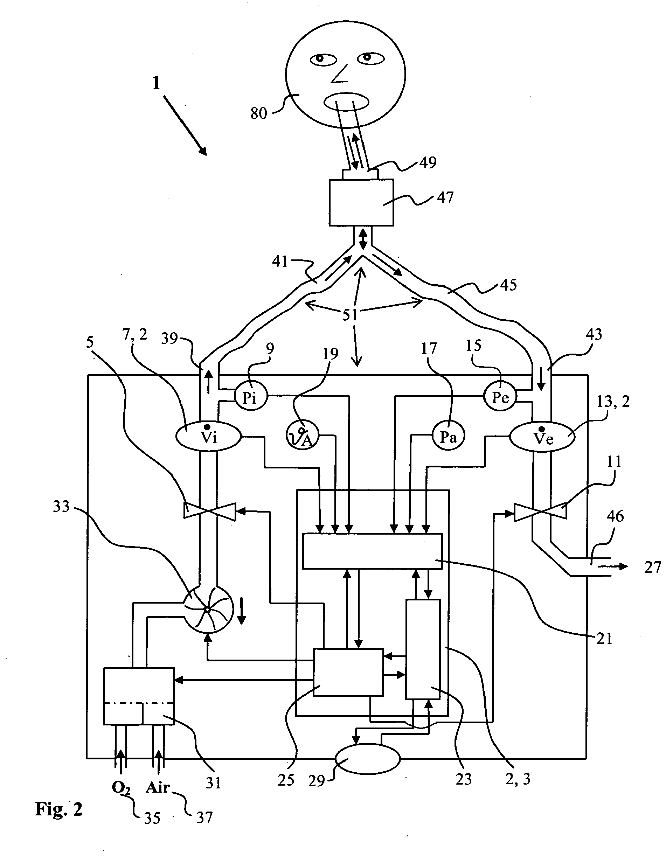

In der Figur 2 ist eine Beatmungsvorrichtung 1 in Verbindung mit einem Patienten 80 als eine Variante einer Fortbildung der medizintechnischen Messvorrichtung 2 nach Figur 1 gezeigt. Gleiche Elemente in den Figuren 1 und 2 sind in der Figur 2 mit den gleichen Bezugsziffern bezeichnet. In dieser Figur 2 ist ein zentrales Betriebs- und Steuerungsmodul 3 vorhanden, welche eine Einheit zur Messwerterfassung, Signalverarbeitung und Berechnung 21 mit Mitteln zur Messwerterfassung, Mitteln zur Signalverarbeitung und Mitteln zur Berechnung, eine Einheit zur Eingabe, Ausgabe und Weiterleitung 23 mit Mitteln zur Eingabe, wie beispielsweise Tast- und Schaltelemente, berührungssensitive Bildschirme Tastaturen, Mitteln zur Ausgabe, wie beispielsweise Leuchtmittel, optische und/ oder akustische Signalmittel, Bildschirme, und eine Datenschnittstelle 29 und eine Kontrolleinheit 25 zur Steuerung- und/oder Regelung umfasst.

Die Beatmungsvorrichtung 1 weist einen inspiratorischen Gasauslass 39 und einen exspiratorischen Gaseinlass 43 auf. Der Patient 80 ist über einen Endotrachialtubus 49 gasführend mittels eines Verbindungsstücks 47, über einen exspiratorischen Beatmungsschlauch 45 mit dem exspiratorischen Gaseinlass 43 verbunden und über einen inspiratorischen Beatmungsschlauch 41 mit dem inspiratorischen Gasauslass 39 verbunden. Weiterhin weist die Beatmungsvorrichtung 1 am inspiratorischen Gasauslass 39 einen inspiratorischen Durchflusssensor 7 und einen inspiratorischen Drucksensor 9, sowie am exspiratorischen Gaseinlass 43 einen exspiratorischen Durchflusssensor 13 und einen exspiratorischen Drucksensor 15 auf. Weitere Sensoren sind ein Umgebungsdrucksensor 17 und ein Umgebungstemperatursensor 19. Die Sensoren 7, 9, 13, 15, 17, 19 sind mit dem Betriebs- und Steuerungsmodul 3 Daten- und Signal- technisch verbunden. Die Einheit zur Messwerterfassung, Signalverarbeitung und Berechnung 21, die Einheit zur Eingabe, Ausgabe und Weiterleitung 23 und die Schnittstelle 29 sind vergleichbar ausgebildet und mit vergleichbaren Funktionalitäten ausgestattet, wie zu der Figur 1 beschrieben. Die Kontrolleinheit 25 ist zusätzlich mit in dem zentralen Betriebs- und Steuerungsmodul 3 angeordnet, um mit Hilfe eines, im inspiratorischen Gasauslass 39 angeordneten und mit der Kontrolleinheit 25 daten- und signaltechnisch verbundenen Inspirations- Dosierventiles 5 und eines, im exspiratorischen Gaseinlass 43 angeordneten und mit der Kontrolleinheit 25 daten- und signaltechnisch verbundenen Exspirationsventiles 11, einen Betrieb der Beatmungsvorrichtung 1 zur Beatmung eines Patienten 80 zu steuern und/oder zu regeln und somit durchzuführen. Weitere Komponenten in der Beatmungsvorrichtung 1 zur Durchführung sind eine Gasmischeinheit 31 und ein Beatmungsantrieb 33. Die Gasmischeinheit 31 mischt mittels einer - in dieser Figur 2 nicht dargestellten Ventil- und Sensorikanordnung - Gase und/ oder Fluide, die der Gasmischeinheit 31 über einen ersten Gasanschluss 35, beispielsweise als Druck- Sauerstoff und über einen zweiten Gasanschluss 37, beispielsweise als medizinische Druckluft Sauerstoff zugeführt werden und stellt diese dem Beatmungsantrieb 33 als frisches Atemgas zur Verfügung. Der Beatmungsantrieb 33 ist vorzugsweise und beispielsweise als ein Faltenbalgantrieb oder als ein Radialverdichter (Blower) ausgeführt. Aus dem Beatmungsantrieb 33 heraus strömt das frische Atemgas während der Einatmung (Inspiration) durch das von der Kontrolleinheit 25 angesteuerte Inspirations-Dosierventil 5 durch den inspiratorischen Durchflusssensor 7 über den inspiratorischen Gasauslass 39 und über einen inspiratorischen Beatmungsschlauch 41, das Verbindungsstück 47 mittels eines Endotrachialtubus 49 oder alternativ einer, in dieser Figur 2 nicht gezeigten Nasalmaske 48, in die Lunge eines Patienten 80. Vom Patienten 80 gelangt durch den Endotrachialtubus 49 oder die Nasalmaske 48 in der Ausatmung (Exspiration) verbrauchtes Atemgas über das Verbindungsstück 47 und einen exspiratorischen Beatmungsschlauch 45 zum exspiratorischen Gaseinlass 43 zurück in die Beatmungsvorrichtung 1, durch den exspiratorischen Durchflusssensor 13 und durch das von der Kontrolleinheit 25 angesteuerte Exspirationsventil 11 schließlich über einen Abluftauslass 46 an eine Umgebung 27 nach außerhalb der Beatmungsvorrichtung 1. Das Verbindungsstück 47, der Endotrachialtubus 49, der inspiratorische Beatmungsschlauch 41, der exspiratorische Beatmungsschlauch 45 bilden mit der Sensorik 7, 9, 13, 15, 17, 19, der Gasmischeinheit 31, dem Beatmungsantrieb 33, dem Exspirationsventil 11, dem Inspirations- Dosierventil 5 und der Kontrolleinheit 25 ein sogenanntes Beatmungssystem 51.

Das Beatmungssystem 51 bildet zusammen mit der medizintechnischen Messvorrichtung 2 (Figur 1) die Beatmungsvorrichtung 1. Die medizintechnische Messvorrichtung 2 (Figur 1) ist in dieser Figur 2 in ein zentrales Betriebs- und Steuerungsmodul 3 eingegliedert. Der inspiratorische Durchflusssensor 7 und der exspiratorische Durchflusssensor 13 sind mit der Einheit zur Messwerterfassung, Signalverarbeitung und Berechnung 21 daten- und signaltechnisch verbunden, in der Einheit zur Messwerterfassung, Signalverarbeitung und Berechnung 21 werden eine, mit dem inspiratorischen Durchflusssensor 7 erfasste, inspiratorische Durchflussmenge und eine mit dem exspiratorischen Durchflusssensor 13 erfasste, exspiratorische Durchflussmenge mittels einer der Formeln 2 oder der Formel 3 basierenden Berechnung in das Maß $S_i$ für eine beatmungsbedingte Verlagerung von Sekret umgerechnet. Dieses Maß $S_i$ wird von der Einheit zur Signalverarbeitung und Berechnung 21 an die Einheit zur Eingabe, Ausgabe und Weiterleitung 23 weitergeleitet, wobei in der Einheit zur Messwerterfassung, Signalverarbeitung und Berechnung 21 auch eine Einordnung des Maßes in einen vorbestimmten Werteraum, z.B. mittels eines Schwellwertvergleiches vorgenommen werden kann. Mit Hilfe der Einheit zur Eingabe, Ausgabe und Weiterleitung 23 wird das Maß $S_i$ direkt, beispielsweise numerisch, grafisch oder in aufbereiteter Form, beispielsweise in Relation zu einem Schwellwert in der Farbgebung adaptiert an den Anwender ausgegeben und/ oder an der Schnittstelle zur Verfügung gestellt. Weiterhin kann das Maß $S_i$ direkt, oder verarbeitet oder umgewandelt an die Kontrolleinheit 25 weitergeleitet werden. Die Kontrolleinheit 25 ist ausgebildet, den Beatmungsantrieb 33 der Beatmungsvorrichtung 1 zur Beatmung eines Patienten 80 auf Basis des Maßes $S_i$ anzupassen.

Eine Anpassung auf Basis des Maßes $S_i$ kann dabei durch eine Variation eines Inspirationsdrucks, eine Variation eines Anstiegs der Druckrampe des Inspirationsdrucks oder einer Anstiegszeitdauer der Druckrampe des Inspirationsdrucks, eine Variation der Inspirations- Durchflussmenge, eine Begrenzung eines Höchstwertes der Inspirations- Durchflussmenge, eine Variation einer inspiratorischen Pause, eine Veränderung einer Beatmungsfrequenz oder eine Veränderung eines I:E-Verhältnisses erfolgen.

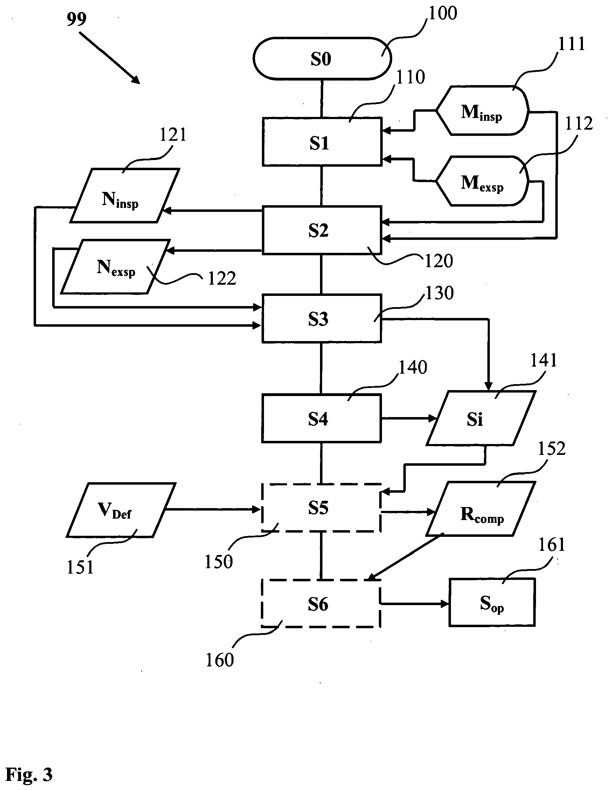

Die Figur 3 zeigt einen schematischen Ablaufplan 99 eines Verfahrens zum Betrieb einer medizintechnischen Messvorrichtung 2 (Figur 1) mit einer inspiratorischen und einer exspiratorischen Durchflussmessung, beispielsweise zum Betrieb der medizintechnischen Messvorrichtung 2 nach Figur 1 oder zum Betrieb der Beatmungsvorrichtung 1 nach Figur 2.

Nach einem Start S0 100 der medizintechnischen Messvorrichtung 2 werden in einem ersten Schritt S1 110 ein inspiratorischer Messwert $M_{insp.}$ 111 und ein exspiratorischer Messwert $M_{exsp.}$ 112 erfasst.

In einem zweiten Schritt S2 120 werden mittels einer mathematischen Beziehung und Integralbildung der inspiratorische Messwert $M_{insp.}$ 111 über ein Integrationsintervall von einer Einatemphase in die inspiratorischen Impulskenngrößen $N_{insp}$, 121 und mittels einer mathematischen Beziehung und Integralbildung der exspiratorische Messwert $M_{exsp.}$ 112 über ein Integrationsintervall von einer Ausatemphase in eine exspiratorische Impulskenngröße $N_{exsp.}$ 122 umgewandelt.

In einem dritten Schritt S3 130 wird aus der inspiratorischen Impulskenngrößen $N_{insp}$ 121 und der exspiratorischen Impulskenngröße $N_{exsp.}$ 122 ein Quotient gebildet und damit das Maß $S_i$ für eine beatmungsbedingte Verlagerung von Sekret 141 bestimmt.

In einem vierten Schritt S4 140 wird das Maß $S_i$ 141 für eine beatmungsbedingte Verlagerung von Sekret ausgegeben oder bereitgestellt.

In einem optimalen fünften Schritt S5 150 wird das Maß $S_i$ 141 mit einem vorbestimmten Wert $V_{Def}$ 151 verglichen und als ein Ergebnis $R_{comp}$ 152 ausgegeben. Das Ergebnis $R_{comp}$ 152 des Vergleiches wird in einem weiteren optimalen sechsten Schritt S6 160 verwertet, um einen Betriebszustand $S_{op}$ 161 der Beatmungsvorrichtung 1 (Figur 2) angepasst an das Maß $S_i$ 141, an den vorbestimmter Wert $V_{Def}$ 151 oder an den Unterschied zwischen dem vorbestimmten Wert $V_{Def}$ 151 und dem Maß $S_i$ 141 einzustellen.

BEZUGSZEICHENLISTE

| | |
|---|---|
| 1 | Beatmungsvorrichtung |
| 2 | Medizintechnische Messeinrichtung |
| 3 | Zentrales Betriebs- und Steuerungsmodul |
| 5 | Inspirations-Dosierventil |
| 7 | Durchflusssensor, inspiratorisch |
| 9 | Drucksensor, inspiratorisch |
| 11 | Exspirationsventil |
| 13 | Durchflusssensor, exspiratorisch |
| 14 | inspir.- und exspiratorischer Durchflusssensor |
| 15 | Drucksensor, exspiratorisch |
| 17 | Umgebungsdrucksensor |
| 19 | Umgebungstemperatursensor |
| 21 | Einheit zur Messwerterfassung, Signalverarbeitung und Berechnung |
| 23 | Einheit zur Eingabe- Ausgabe- und Weiterleitung |
| 25 | Kontrolleinheit |
| 27 | Umgebung |
| 29 | Schnittstelle |
| 31 | Gasmischeinheit |
| 33 | Beatmungsantrieb |
| 35 | erster Gasanschluss |
| 37 | zweiter Gasanschluss |
| 39 | inspiratorischer Gasauslass, |
| 41 | inspiratorischer Beatmungsschlauch |

| | |
|---|---|
| 43 | exspiratorischer Gaseinlass, |
| 45 | exspiratorischer Beatmungsschlauch |
| 46 | Abluftauslass |
| 47 | Verbindungsstück, Y-Stück |
| 48 | Nasalmaske |
| 49 | Endotrachialtubus |
| 50 | Mundstück |
| 80 | Patient |
| 81, 82, 83 | Darstellungsvarianten der Medizintechnische Messeinrichtung 2 |
| 99 | Ablaufplan |
| 100 | Start S0 |
| 110 | Erster Schritt S1 |
| 111 | inspiratorischer Messwert $M_{insp.}$ |
| 112 | exspiratorischer Messwert $M_{exsp.}$ |
| 120 | Zweiter Schritt S2 |
| 111 | inspiratorische Impulskenngröße $N_{insp.}$ |
| 112 | exspiratorische Impulskenngröße $N_{exsp.}$ |
| 130 | Dritter Schritt S3 |
| 140 | Vierter Schritt S4 |
| 141 | Maß $S_i$ für eine beatmungsbedingte Verlagerung von Sekret |
| 150 | Fünfter Schritt S5 |
| 151 | Vorbestimmter Wert $V_{Def}$ |
| 152 | Ergebnis $R_{comp}$ |
| 160 | Sechster Schritt S6 |
| 161 | Betriebszustand $S_{op}$ |

**Patentansprüche**

1. Medizintechnische Messvorrichtung (2) mit Mitteln zur Messwerterfassung, Signalverarbeitung und Berechnung (21) und mit einer Sensorik (7, 9, 13, 14, 15, 17, 19), welche ausgebildet ist,
eine inspiratorische Messgröße (111) zu erfassen, welche ein Maß für einen Transport von Atemgasen in eine Lunge eines Patienten (80) hinein darstellt und
eine exspiratorische Messgröße (112) zu erfassen, welche ein Maß für einen Transport von Atemgasen aus der Lunge eines Patienten (80) hinaus darstellt,
**dadurch gekennzeichnet, dass**
die Mittel zur Messwerterfassung, Signalverarbeitung und Berechnung (21) dazu ausgebildet sind, aus einem Quotienten aus der inspiratorischen Messgröße (111) und der exspiratorischen Messgröße (112) ein Maß (141) für eine beatmungsbedingte Verlagerung von Sekret zu bestimmen.

2. Medizintechnische Messvorrichtung (2) nach Anspruch 1, wobei die Mittel zur Messwerterfassung, Signalverarbeitung und Berechnung (21) dazu ausgebildet sind, einen Vergleich des Maßes (141) für die beatmungsbedingte Verlagerung von Sekret mit einem vorbestimmten Wert (151) durchzuführen.

3. Medizintechnische Messvorrichtung (2) nach Anspruch 1 oder Anspruch 2 mit Mitteln zur Eingabe, Ausgabe und Weiterleitung (23), wobei die Mittel zur Eingabe, Ausgabe und Weiterleitung (23) ausgebildet sind, das Maß (141) für eine beatmungsbedingte Verlagerung von Sekret, ein Ergebnis des Vergleichs zwischen dem Maß (141) für die beatmungsbedingte Verlagerung von Sekret mit einem vorbestimmten Wert (151) oder einen Hinweis bezüglich einer Relation des Maßes der beatmungsbedingten Verlagerung zum vorbestimmten Wert (151) auszugeben, anzuzeigen oder weiterzuleiten.

4. Medizinische Messvorrichtung (2) nach einem der vorhergehenden Ansprüche, wobei die Sensorik (7, 9, 13, 14, 15, 17, 19) zur Erfassung der inspiratorischen Messgröße (111) ein inspiratorischer Durchflusssensor (7) oder ein inspiratorischer Drucksensor (9) ist.

5. Medizinische Messvorrichtung (2) nach einem der vorhergehenden Ansprüche, wobei die Sensorik (7, 9, 13, 14, 15, 17, 19) zur Erfassung der exspiratorischen Messgröße (112) ein exspiratorischer Durchflusssensor (13) oder ein exspiratorischer Drucksensor (15) ist.

6. Medizinische Messvorrichtung (2) nach einem der vorhergehenden Ansprüche, wobei die Sensorik (7, 9, 13, 14, 15, 17, 19) zur Erfassung der inspiratorischen Messgröße (111) und zur Erfassung der exspiratorischen Messgröße (112) ein in- und exspiratorischer Durchflusssensor (14) oder ein patientennaher in- und exspiratorischer Durchflusssensor (14) ist.

7. Beatmungsvorrichtung (1) mit einem Beatmungssystem (51) und mit einer medizintechnischen Messvorrichtung (2) nach einem der Ansprüche 1 bis 6, wobei in oder an dem Beatmungssystem (51) eine Aktuatorik (5, 11, 33) zu einem Transport von Atemgasen in eine Lunge eines Patienten (80) hinein und aus der Lunge eines Patienten (80) hinaus angeordnet ist, wobei in oder an dem Beatmungssystem (51) eine Kontrolleinheit (25) zu einer Steuerung eines Betriebes eines Beatmungssystems (51) und zu einer Steuerung und/oder einer Regelung einer Beatmung angeordnet ist, wobei in oder an dem Beatmungssystem (51) Mittel zur Eingabe, Ausgabe und Weiterleitung (23) zu einer Bedienung des Beatmungssystems (51) oder der Beatmungsvorrichtung (1) und/oder zu einem Datenaustausch mit anderen Geräten oder Systemen angeordnet sind und wobei in oder an dem Beatmungssystem (51) Verbindungsmittel (41, 43) zu einer pneumatischen, gasführenden Verbindung des Beatmungssystems (51) mit einem Patienten (80) angeordnet sind.

8. Beatmungsvorrichtung (1) nach Anspruch 7, wobei die Mittel zur Messwerterfassung, Signalverarbeitung und Berechnung (21) oder die Kontrolleinheit (25) ausgebildet sind, das Maß für die beatmungsbedingte Verlagerung von Sekret (141) mit einem vorbestimmten Wert zu vergleichen.

9. Beatmungsvorrichtung (1) nach Anspruch 7 oder nach Anspruch 8, wobei die Mittel zur Eingabe, Ausgabe und Weiterleitung (23) ausgebildet sind, das Maß für die beatmungsbedingte Verlagerung von Sekret (141), ein Ergebnis des Vergleichs zwischen dem Maß für die beatmungsbedingte Verlagerung von Sekret mit einem vorbestimmten Wert oder einen Hinweis bezüglich einer Relation des Maßes der beatmungsbedingten Verlagerung zum vorbestimmten Wert, weiterzuleiten oder an einen Anwender auszugegeben.

10. Beatmungsvorrichtung (1) nach einem der Ansprüche 7 bis 9, wobei die Kontrolleinheit (25) ausgebildet ist, bei einer Abweichung des Maßes der beatmungsbedingten Verlagerung von Sekret vom vorbestimmten Wert, einen Stellwert eines Inspirationsdrucks zu verändern oder anzupassen, einen Stellwert einer Inspirations-Durchflussmenge zu verändern oder anzupassen, einen Höchstwert einer Inspirations-Durchflussmenge zu verändern oder anzupassen, einen Anstieg einer Druckrampe des Inspirationsdrucks zu verändern oder anzupassen, eine Anstiegszeitdauer der Druckrampe des Inspirationsdrucks zu verändern oder anzupassen, eine inspiratorische Pause zu verändern oder anzupassen, ein Inspirations- zu Exspirationsverhältnis oder eine Beatmungsfrequenz zu verändern oder anzupassen.

11. Beatmungsvorrichtung (1) nach einem der Ansprüche 7 bis 10, wobei die Kontrolleinheit (25) in Zusammenwirkung mit der Aktuatorik (5, 11, 33) ausgebildet ist, bei einer Überschreitung des vorbestimmten Wertes durch das Maß der beatmungsbedingten Verlagerung von Sekret (141), einen Stellwert eines Inspirationsdrucks zu verringern, einen Stellwert einer Inspirations-Durchflussmenge zu verringern, einen Höchstwert einer Inspirations-Durchflussmenge zu begrenzen, einen Anstieg einer Druckrampe des Inspirationsdrucks zu verringern oder eine Anstiegszeitdauer der Druckrampe des Inspirationsdrucks zu verlängern oder eine inspiratorische Pause zu verlängern, so dass das Maß der beatmungsbedingten Verlagerung von Sekret (141) den vorbestimmten Wert in der Folge wieder unterschreitet.

12. Beatmungsvorrichtung (1) nach einem der Ansprüche 7 bis 11, wobei die Kontrolleinheit (25) in Zusammenwirkung mit der Aktuatorik (5, 11, 33) ausgebildet ist, bei einer Überschreitung des vorbestimmten Wertes (151) durch das Maß der beatmungsbedingten Verlagerung von Sekret (141), ein Inspirations- zu Exspirationsverhältnis und/oder eine Beatmungsfrequenz zu verändern oder anzupassen, so dass das Maß der beatmungsbedingten Verlagerung von Sekret (141) den vorbestimmten Wert in der Folge wieder unterschreitet.

13. Verfahren zum Betrieb einer medizintechnischen Messvorrichtung (2) nach einem Ansprüche 1 bis 6, wobei in einem ersten Schritt (110) mittels der Sensorik (7, 9, 13, 14, 15, 17, 19) und mit den Mitteln zur Messwerterfassung, Signalverarbeitung und Berechnung (21) eine inspiratorische Messgröße (111) und eine exspiratorische Messgröße (112) erfasst werden, in einem zweiten Schritt (120) mit den Mitteln zur Messwerterfassung, Signalverarbeitung und Berechnung (21) die inspiratorische Messgröße (111) mittels einer ersten progressiven mathematischen Beziehung in eine inspiratorische Impulskenngröße (121) und die exspiratorische Messgröße (112) mittels einer zweiten progressiven mathematischen Beziehung in eine exspiratorische Impulskenngröße (122) mathematisch umgewandelt werden, in einem dritten Schritt (130) mit den Mitteln zur Messwerterfassung, Signalverarbeitung und Berechnung (21) ein Quotient aus der inspiratorischen Impulskenngröße (121) und der exspiratorischen Impulskenngröße (122) als ein Maß für eine beatmungsbedingte Verlagerung von Sekret (141) gebildet wird.

14. Verfahren nach Anspruch 13, wobei in einem weiteren Schritt mit den Mitteln zur Eingabe, Ausgabe und Weiterleitung (23) das Maß für die beatmungsbedingte Verlagerung von Sekret (141) ausgegeben oder weitergeleitet wird.

15. Verfahren nach Anspruch 13 oder Anspruch 14, wobei in einem weiteren Schritt mit der Einheit zur Messwerterfassung, Signalverarbeitung und Berechnung (21) das Maß für die beatmungsbedingte Verlagerung von Sekret (141) mit einem vorbestimmten Wert (151) verglichen wird und mittels der Mittel zur Eingabe, Ausgabe und Weiterleitung (23) ein Hinweis (152) bezüglich einer Relation des Maßes der beatmungsbedingten Verlagerung zum vorbestimmten Wert weitergeleitet oder an einen Anwender ausgegeben wird.

16. Verwendung einer medizinischen Messvorrichtung (2) nach einem der Ansprüche 1 bis 6, wie auch einem der Ansprüche 7 bis 12, wobei mit den Mitteln zur Eingabe, Ausgabe und Weiterleitung (23)

- eine Bestätigung für mindestens einen Vorschlagswert angefordert wird,
- ein bestätigungsspezifisches Signal erfasst wird,
- das bestätigungsspezifische Signal zu einem Steuersignal verarbeitet wird und
- das Steuersignal an eine Kontrolleinheit (25)
der Beatmungsvorrichtung (1) bereitgestellt wird.

**Claims**

1. A medical measuring device (2) having means for measured-value detection, signal-processing and calculation (21) and having a sensor system (7, 9, 13, 14, 15, 17, 19), which is configured
to detect an inspiratory measured variable (111) that represents a measure of the transportation of respiratory gases into the lungs of a patient (80), and to detect an expiratory measured variable (112) that represents a measure of the transportation of respiratory gases out of the lungs of a patient (80),
**characterised in that**
the means for measured-value detection, signal-processing and calculation (21) are configured to determine from a quotient of the inspiratory measured variable (111) and the expiratory measured variable (112) a measure (141) of a respiration-related shift of secretion.

2. A medical measuring device (2) according to claim 1,
wherein the means for measured-value detection, signal-processing and calculation (21) are configured to carry out a comparison of the measure (141) of the respiration-related shift of secretion with a predetermined value (151).

3. A medical measuring device (2) according to claim 1 or claim 2 having means for inputting, outputting and forwarding (23), wherein the means for inputting, outputting and forwarding (23) are configured to output, display or forward the measure (141) of a respiration-related shift of secretion, a result of the comparison between the measure (141) of the respiration-related shift of secretion with a predetermined value (151), or an indication of a relationship of the measure of the respiration-related shift with respect to the predetermined value (151).

4. A medical measuring device (2) according to one of the preceding claims, wherein the sensor system (7, 9, 13, 14, 15, 17, 19) for the detection of the inspiratory measured variable (111) is an inspiratory flow sensor (7) or an inspiratory pressure sensor (9).

5. A medical measuring device (2) according to one of the preceding claims, wherein the sensor system (7, 9, 13, 14, 15, 17, 19) for the detection of the expiratory measured variable (112) is an expiratory flow sensor (13) or an expiratory pressure sensor (15).

6. A medical measuring device (2) according to one of the preceding claims, wherein the sensor system (7, 9, 13, 14, 15, 17, 19) for the detection of the inspiratory measured variable (111) and for the detection of the expiratory measured variable (112) is an inspiratory and expiratory flow sensor (14) or an inspiratory and expiratory flow sensor (14) close to the patient.

7.  A respiratory device (1) having a respiratory system (51) and having a medical measuring device (2) according to one of claims 1 to 6, wherein arranged in or at the respiratory system (51) there is an actuator mechanism (5, 11, 33) for transportation of respiratory gases into the lungs of a patient (80) and out of the lungs of a patient (80), wherein arranged in or at the respiratory system (51) there is a checking unit (25) for controlling an operation of a respiratory system (51) and for controlling and/or regulating respiration, wherein arranged in or at the respiratory system (51) there are means for inputting, outputting and forwarding (23) for operation of the respiratory system (51) or the respiratory device (1) and/or for data-exchange with other equipment or systems, and wherein arranged in or at the respiratory system (51) there are connection means (41, 43) for pneumatic, gas-carrying connection of the respiratory system (51) to a patient (80).

8.  A respiratory device (1) according to claim 7, wherein the means for measured-value detection, signal-processing and calculation (21) or the checking unit (25) are configured to compare the measure of the respiration-related shift of secretion (141) with a predetermined value.

9.  A respiratory device (1) according to claim 7 or according to claim 8, wherein the means for inputting, outputting and forwarding (23) are configured to forward or output to a user the measure of the respiration-related shift of secretion (141), a result of the comparison between the measure (141) of the respiration-related shift of secretion with a predetermined value, or an indication of a relationship of the measure of the respiration-related shift with respect to the predetermined value.

10. A respiratory device (1) according to one of claims 7 to 9, wherein the checking unit (25) is configured to change or adapt a set value of an inspiratory pressure, to change or adapt a set value of an inspiratory flow rate, to change or adapt a maximum value of an inspiratory flow rate, to change or adapt a rise in a pressure ramp of the inspiratory pressure, to change or adapt the duration of a rise of the pressure ramp of the inspiratory pressure, to change or adapt an inspiratory pause, to change or adapt an inspiration to expiration ratio or a respiration frequency, in the event of a deviation of the measure of the respiration-related shift of secretion from the predetermined value.

11. A respiratory device (1) according to one of claims 7 to 10, wherein the checking unit (25) is configured, in conjunction with the actuator mechanism (5, 11, 33), to reduce a set value of an inspiratory pressure, to reduce a set value of an inspiratory flow rate, to limit a maximum value of an inspiratory flow rate, to reduce a rise of a pressure ramp of the inspiratory pressure or to lengthen the duration of a rise of the pressure ramp of the inspiratory pressure or to lengthen an inspiratory pause, in the event of the measure of the respiration-related shift of secretion (141) exceeding the predetermined value, so that the measure of the respiration-related shift of secretion (141) falls back below the predetermined value as a result.

12. A respiratory device (1) according to one of claims 7 to 11, wherein the checking unit (25) is configured, in conjunction with the actuator mechanism (5, 11, 33), to change or adapt an inspiration to expiration ratio and/or a respiration frequency, in the event of the measure of the respiration-related shift of secretion (141) exceeding the predetermined value (151), so that the measure of the respiration-related shift of secretion (141) falls back below the predetermined value as a result.

13. A method for operating a medical measuring device (2) according to one of claims 1 to 6, wherein
    in a first step (110) by means of the sensor system (7, 9, 13, 14, 15, 17, 19) and with the means for measured-valued detection, signal-processing and calculation (21) an inspiratory measured variable (111) and an expiratory measured variable (112) are detected,
    in a second step (120) with the means for measured-value detection, signal-processing and calculation (21) the inspiratory measured variable (111) is mathematically converted by means of a first progressive mathematical relationship into an inspiratory pulse parameter (121) and the expiratory measured variable (112) is mathematically converted by means of a second progressive mathematical relationship into an expiratory pulse parameter (122),
    in a third step (130) with the means for measured-valued detection, signal-processing and calculation (21) a quotient is formed from the inspiratory pulse parameter (121) and the expiratory pulse parameter (122) as a measure of a respiration-related shift of secretion (141).

14. A method according to claim 13, wherein in a further step with the means for inputting, outputting and forwarding (23) the measure of the respiration-related shift of secretion (141) is output or forwarded.

15. A method according to claim 13 or claim 14, wherein in a further step with the unit for measured-value detection, signal-processing and calculation (21) the measure of the respiration-related shift of secretion (141) is compared with a predetermined value (151) and by means of the means for inputting, outputting and forwarding (23) an indication (152) of a relationship of the measure of the respiration-related shift with respect to the predetermined value is forwarded or output to a user.

16. Use of a medical measuring device (2) according to one of claims 1 to 6, as well as one of claims 7 to 12, wherein with the means for inputting, outputting and forwarding (23)

- confirmation for at least one proposed value is requested,
- a confirmation-specific signal is detected,
- the confirmation-specific signal is processed into a control signal, and
- the control signal is made available to a checking unit (25) of the respiratory device (1).

**Revendications**

1. Dispositif de mesure médico-technique (2) comprenant des moyens (21) d'acquisition de valeurs mesurées, de traitement de signaux et de calcul, et un ensemble de détection (7, 9, 13, 14, 15, 17, 19) réalisé en vue de saisir une grandeur de mesure inspiratoire (111), représentant un critère de mesure d'une introduction de gaz respiratoires dans un poumon d'un patient (80), et de saisir une grandeur de mesure expiratoire (112), représentant un critère de mesure d'une expulsion de gaz respiratoires hors du poumon d'un patient (80), **caractérisé par le fait que** les moyens (21) d'acquisition de valeurs mesurées, de traitement de signaux et de calcul sont réalisés en vue de déterminer, sur la base d'un quotient de la grandeur de mesure inspiratoire (111) et de la grandeur de mesure expiratoire (112), un critère de mesure (141) d'un déplacement de sécrétions conditionné par la ventilation.

2. Dispositif de mesure médico-technique (2) selon la revendication 1, les moyens (21) d'acquisition de valeurs mesurées, de traitement de signaux et de calcul étant réalisés en vue d'effectuer une comparaison, avec une valeur prédéterminée (151), du critère de mesure (141) du déplacement de sécrétions conditionné par la ventilation.

3. Dispositif de mesure médico-technique (2) selon la revendication 1 ou la revendication 2, équipé de moyens (23) d'entrée, de sortie et de transmission, lesdits moyens (23) d'entrée, de sortie et de transmission étant réalisés en vue de délivrer, d'afficher ou de transmettre le critère de mesure (141) d'un déplacement de sécrétions conditionné par la ventilation ; un résultat de la comparaison, avec une valeur prédéterminée (151), dudit critère de mesure (141) du déplacement de sécrétions conditionné par la ventilation ; ou une indication ayant trait à une relation existant entre ladite valeur prédéterminée (151) et ledit critère de mesure dudit déplacement conditionné par la ventilation.

4. Dispositif de mesure médico-technique (2) selon l'une des revendications précédentes, l'ensemble de détection (7, 9, 13, 14, 15, 17, 19), affecté à la saisie de la grandeur de mesure inspiratoire (111), étant un capteur (7) de débit inspiratoire ou un capteur (9) de pression inspiratoire.

5. Dispositif de mesure médico-technique (2) selon l'une des revendications précédentes, l'ensemble de détection (7, 9, 13, 14, 15, 17, 19), affecté à la saisie de la grandeur de mesure expiratoire (112), étant un capteur (13) de débit expiratoire ou un capteur (15) de pression expiratoire.

6. Dispositif de mesure médico-technique (2) selon l'une des revendications précédentes, l'ensemble de détection (7, 9, 13, 14, 15, 17, 19), affecté à la saisie de la grandeur de mesure inspiratoire (111) et à la saisie de la grandeur de mesure expiratoire (112), étant un capteur (14) de débits inspiratoire et expiratoire, ou un capteur (14) de débits inspiratoire et expiratoire, placé à proximité du patient.

7. Dispositif respiratoire (1) équipé d'un système de respiration (51) et d'un dispositif de mesure médico-technique (2) conforme à l'une des revendications 1 à 6, un ensemble d'actionnement (5, 11, 33) étant implanté dans ou sur ledit système de respiration (51), en vue d'une introduction de gaz respiratoires dans un poumon d'un patient (80) et de leur expulsion hors du poumon d'un patient (80), sachant qu'une unité de contrôle (25), dévolue à une commande d'un fonctionnement d'un système de respiration (51), et à une commande et/ou à une régulation d'une ventilation, est implantée dans ou sur ledit système de respiration (51), sachant que des moyens (23) d'entrée, de sortie et de transmission, dévolus à une manoeuvre du système de respiration (51) ou du dispositif respiratoire (1), et/ou à un échange de données avec d'autres appareils ou systèmes, sont implantés dans ou sur ledit système de respiration (51), et sachant que des moyens de liaison (41, 43), destinés à relier pneumatiquement le système de respiration (51) à un patient (80), avec effet de guidage des gaz, sont implantés dans ou sur ledit système de respiration (51).

8. Dispositif respiratoire (1) selon la revendication 7, les moyens (21) d'acquisition de valeurs mesurées, de traitement de signaux et de calcul, ou l'unité de contrôle (25), étant réalisé(e)(s) en vue de comparer, avec une valeur prédéterminée, le critère de mesure (141) du déplacement de sécrétions conditionné par la ventilation.

9. Dispositif respiratoire (1) selon la revendication 7 ou la revendication 8, les moyens (23) d'entrée, de sortie et de transmission étant réalisés en vue de transmettre, ou de notifier à un utilisateur, le critère de mesure (141) du déplacement de sécrétions conditionné par la ventilation ; un résultat de la comparaison, avec une valeur prédéterminée, dudit critère de mesure du déplacement de sécrétions conditionné par la ventilation ; ou une indication ayant trait à une relation existant entre ladite valeur prédéterminée et ledit critère de mesure dudit déplacement conditionné par la ventilation.

10. Dispositif respiratoire (1) selon l'une des revendications 7 à 9, sachant que, en présence d'un écart entre la valeur prédéterminée et le critère de mesure du déplacement de sécrétions conditionné par la ventilation, l'unité de contrôle (25) est réalisée en vue de modifier ou d'adapter une valeur de réglage d'une pression inspiratoire, de modifier ou d'adapter une valeur de réglage d'un débit inspiratoire, de modifier ou d'adapter une valeur maximale d'un débit inspiratoire, de modifier ou d'adapter un accroissement d'une rampe de la pression inspiratoire, de modifier ou d'adapter une durée d'accroissement de la rampe de ladite pression inspiratoire, de modifier ou d'adapter une pause inspiratoire, de modifier ou d'adapter un rapport entre l'inspiration et l'expiration, voire une fréquence de ventilation.

11. Dispositif respiratoire (1) selon l'une des revendications 7 à 10, sachant que, dans le cas où le critère de mesure (141) du déplacement de sécrétions conditionné par la ventilation excède la valeur prédéterminée, l'unité de contrôle (25) est réalisée pour provoquer, en coopération avec l'ensemble d'actionnement (5, 11, 33), la diminution d'une valeur de réglage d'une pression inspiratoire, la diminution d'une valeur de réglage d'un débit inspiratoire, la limitation d'une valeur maximale d'un débit inspiratoire, la diminution d'un accroissement d'une rampe de la pression inspiratoire ou l'allongement d'une durée d'accroissement de la rampe de ladite pression inspiratoire, voire la prolongation d'une pause inspiratoire, de telle sorte que, à un stade ultérieur, ledit critère de mesure (141) du déplacement de sécrétions conditionné par la ventilation excède, de nouveau, négativement ladite valeur prédéterminée.

12. Dispositif respiratoire (1) selon l'une des revendications 7 à 11, sachant que, dans le cas où le critère de mesure (141) du déplacement de sécrétions conditionné par la ventilation excède la valeur prédéterminée (151), l'unité de contrôle (25) est réalisée pour provoquer, en coopération avec l'ensemble d'actionnement (5, 11, 33), une modification ou une adaptation d'un rapport entre l'inspiration et l'expiration, et/ou d'une fréquence de ventilation, de telle sorte que, à un stade ultérieur, ledit critère de mesure (141) dudit déplacement de sécrétions conditionné par la ventilation excède, de nouveau, négativement ladite valeur prédéterminée.

13. Procédé d'actionnement d'un dispositif de mesure médico-technique (2) conforme à l'une des revendications 1 à 6, dans lequel,
au cours d'une première étape (110), une grandeur de mesure inspiratoire (111) et une grandeur de mesure expiratoire (112) sont saisies au moyen de l'ensemble de détection (7, 9, 13, 14, 15, 17, 19) et à l'aide des moyens (21) d'acquisition de valeurs mesurées, de traitement de signaux et de calcul,
au cours d'une deuxième étape (120), à l'aide desdits moyens (21) d'acquisition de valeurs mesurées, de traitement de signaux et de calcul, ladite grandeur de mesure inspiratoire (111) et ladite grandeur de mesure expiratoire (112) sont respectivement converties mathématiquement en une grandeur caractéristique d'impulsion inspiratoire (121), au moyen d'une première relation mathématique progressive, et en une grandeur caractéristique d'impulsion expiratoire (122), au moyen d'une seconde relation mathématique progressive,
au cours d'une troisième étape (130), un quotient de ladite grandeur caractéristique d'impulsion inspiratoire (121) et de ladite grandeur caractéristique d'impulsion expiratoire (122) est formé, à l'aide desdits moyens (21) d'acquisition de valeurs mesurées, de traitement de signaux et de calcul, en tant que critère de mesure (141) d'un déplacement de sécrétions conditionné par la ventilation.

14. Procédé selon la revendication 13, dans lequel, au cours d'une étape supplémentaire, le critère de mesure (141) du déplacement de sécrétions conditionné par la ventilation est délivré ou transmis à l'aide des moyens (23) d'entrée, de sortie et de transmission.

**15.** Procédé selon la revendication 13 ou la revendication 14, dans lequel, au cours d'une étape supplémentaire, le critère de mesure (141) du déplacement de sécrétions conditionné par la ventilation est comparé à une valeur prédéterminée (151), au moyen de l'unité (21) d'acquisition de valeurs mesurées, de traitement de signaux et de calcul, et une indication (152), ayant trait à une relation existant entre ladite valeur prédéterminée et ledit critère de mesure dudit déplacement conditionné par la ventilation, est transmise ou notifiée à un utilisateur à l'aide des moyens (23) d'entrée, de sortie et de transmission.

**16.** Utilisation d'un dispositif de mesure médico-technique (2) conforme à l'une des revendications 1 à 6, ainsi qu'à l'une des revendications 7 à 12, les moyens (23) d'entrée, de sortie et de transmission effectuant

- la demande d'une confirmation relative pour au moins une valeur proposée,
- la saisie d'un signal spécifique de la confirmation,
- le traitement dudit signal spécifique de ladite confirmation, pour obtenir un signal de commande, et
- l'application dudit signal de commande à une unité de contrôle (25) du dispositif respiratoire (1).

**Fig. 1**

**Fig. 2**

**Fig. 3**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2904035 A
- US 5400777 A
- US 5937853 A
- WO 2007085110 A1
- WO 2008098382 A1
- US 6571792 B
- US 6553990 B
- WO 2010022513 A1
- US 5606968 A
- WO 2010058308 A2

**In der Beschreibung aufgeführte Nicht-Patentliteratur**

- *Respiratory Care,* Oktober 2008, vol. 53 (10